# EUROPÄISCHE PATENTSCHRIFT

(11) **EP 3 558 629 B1**
(45) Veröffentlichungstag und Bekanntmachung des Hinweises auf die Patenterteilung: **15.11.2023**
(21) Anmeldenummer: 17825472.8
(22) Anmeldetag: 15.12.2017
(51) Int. Cl.: B29C 48/685, B29C 48/395, B29C 48/535, B29C 48/63

(54) **EINWELLENEXTRUDER UND VERWENDUNG EINES EINWELLENEXTRUDERS SOWIE VERFAHREN ZUM ÄNDERN EINER MORPHOLOGIE EINES SUPERABSORBIERENDEN POLYMERGELS MIT EINEM EINWELLENEXTRUDER**
SINGLE-SHAFT EXTRUDER AND USE OF A SINGLE-SHAFT EXTRUDER, AND METHOD FOR ALTERING MORPHOLOGY OF A SUPERABSORBENT POLYMER GEL USING A SINGLE-SHAFT EXTRUDER
EXTRUDEUSE MONOVIS ET SON UTILISATION ET PROCÉDÉ POUR MODIFIER LA MORPHOLOGIE D'UN GEL POLYMÈRE SUPERABSORBANT À L'AIDE D'UNE EXTRUDEUSE MONOVIS

(30) Priorität: 21.12.2016 EP 16205993
(43) Veröffentlichungstag der Anmeldung: 30.10.2019
(73) Patentinhaber: BASF SE, 67056 Ludwigshafen am Rhein (DE)
(72) Erfinder: HAAG, Monica, 67056 Ludwigshafen (DE); DEUERLEIN, Stephan, 67056 Ludwigshafen (DE); BARTHEL, Holger, 67056 Ludwigshafen (DE); KLOCK, Volker, 67056 Ludwigshafen (DE); KRAUSS, Roland, 67056 Ludwigshafen (DE)
(74) Vertreter: BASF IP Association
(86) Internationale Anmeldenummer: PCT/EP2017/083107
(87) Internationale Veröffentlichungsnummer: WO 2018/114703

(56) Entgegenhaltungen:
- EP-A1- 0 490 361
- EP-A1- 2 208 756
- EP-A2- 0 209 328
- DE-A1- 2 235 784
- JP-A- H1 177 667
- JP-A- H04 353 424
- JP-A- 2005 001 231

## Beschreibung

Die vorliegende Erfindung betrifft einen Einwellenextruder nach dem Oberbegriff des Anspruchs 1 zum Ändern einer Morphologie von superabsorbierendem Polymergel (SAP-Polymergel) sowie eine Verwendung desselben, insbesondere zur Granulierung des superabsorbierenden Polymergels (SAP-Polymergels), und ein Verfahren zum Ändern einer Morphologie eines SAP-Polymergels mit dem Einwellenextruder.

In WO 2014/118024 A1 ist ein Verfahren zur Herstellung wasserabsorbierender Polymerpartikel mit hoher Quellgeschwindigkeit und hoher Zentrifugenretentionskapazitat bei gleichzeitig hoher Permeabilität des gequollenen Gelbetts durch Polymerisation einer wässrigen Monomerlösung in einem Polymerisationsreaktor mit mindestens zwei achsparallel rotierenden Wellen (Kneter) beschrieben. Ein daraus hervorgehendes Polymergel wird anschließend einer Extrusion bei hohen Temperaturen und thermischer Oberflächennachvemetzung unterworfen. Das Polymergel weist während der Extrusion eine Temperatur größer 80°C auf und es wird bei der Extrusion weniger als 60 kWh/t an spezifischer mechanischer Energie eigetragen. Die bei der Extrusion eingetragene spezifische mechanische Energie (SME) kann beispielsweise über das Verhältnis von Innenlänge zu Innendurchmesser des Extruders (L/D) beeinflusst werden. Die spezifische mechanische Energie (SME) ist die Motorleistung des Extruders in kW dividiert durch den Durchsatz an Polymergel in t/h. Es werden vorteilhaft kurze Extruder eingesetzt. Dadurch werden zu hohe Drücke bei der Extrusion vermieden. Bei der Extrusion wird das Polymergel durch die Löcher einer Lochplatte gedrückt. Die Eigenschaften wasserabsorbierender Polymerpartikel lassen sich verbessern, wenn im Polymerisationsreaktor mit mindestens zwei achsparallel rotierenden Wellen hergestellte höher vernetzte Polymergele zusätzlich bei höheren Temperaturen extrudiert werden. Die dabei auftretenden Scherkräfte führen zu raueren Polymerpartikeln, die nach der thermischen Oberflächen-nachvernetzung eine hohe Quellgeschwindigkeit (FSR) und eine hohe Zentrifugenretentionskapazität (CRC) aufweisen. Zu hohe Energieeinträge bei der Extrusion führen allerdings zu einer Verschlechterung der Quellgeschwindigkeit (FSR) und der Zentrifugenretentionskapazität (CRC) und sind daher zu meiden. Die aus der Patentanmeldung WO 2014/118024 A1 bekannte Lehre und der Inhalt der WO 2014/118024 A1 sind Bestandteil dieses Textes und sind unter Bezugnahme auf die WO 2014/118024 A1 hier aufgenommen.

EP 2 208 756 A1 beschreibt --zur Verwendung in Absorptionsartikeln wie z.B. Papierwindeln, Damenbinden und Inkontinenzeinlagen-- die Herstellung eines wasserabsorbierenden Harzes, wobei eine Zusammensetzung das wasserabsorbierende Harz als Hauptbestandteil enthält, mit hervorragender deodoranten und antimikrobiellen Eigenschaften ohne Beeinträchtigung von Aussehen und Saugfähigkeit des wasserabsorbierenden Harzes.

Unter anderem wird dabei ein Gel gemahlen, was bei der Polymerisation oder nach der Polymerisation durchgeführt wird, und vorzugsweise wird das Gel durch Extrudieren gemahlen mit einem kontinuierlichen Kneter oder einer porösen Struktur.

Als Extrusions-Mahl-Vorrichtung kann beispielsweise ein Vorrichtungstyp verwendet werden, der in der Lage ist, das wasserhaltige Gel unter Druck von einer Zuführöffnung zu einer porösen Platte zu fördern; wie beispielsweise ein Extruder vom Schraubentyp oder Rotationswalzentyp. Ein Schraubenextruder kann einachsig oder mehr-achsig sein und kann normalerweise beim Extrudieren von Fleisch, Gummi oder Kunststoff, oder als Schleifmaschine verwendet werden. Zum Beispiel kann ein Fleischhacker oder eine Kuppelmühle oder dergleichen vorhanden sein,

Aus EP 2 208 756 A1 ist es bevorzugt, dass ein wasserhaltiges Gel nach dem Trocknen so eingestellt wird, dass es eine spezifische Partikelgröße hat, um Eigenschaften bei einer Oberflächenvernetzung zu unterstützen.

In JP 2005 001231 A wird ein Gummi kontinuierlich knetender Extruder bereitgestellt, der in der Lage ist, ein Gummi ausreichend zu kneten und den aufgrund der Wärmeerzeugung auftretenden Temperaturanstieg im Gummi zu unterdrücken. Bei dem Gummi-Durchlaufknetextruder ist eine Gummizuführung zum Aufnehmen des Vorrats eines Gummimaterials, eine Schnecke, und ein Zylinder angeordnet am Außenumfang der Schnecke, und ein Kühlteil in mindestens einem Abschnitt des Zylinders oder der Schnecke vorgesehen. Die Schnecke ist mit einer Schneckenwelle und einer Vielzahl von Schneckenelementen entlang der Schneckenwelle ausgeführt. Für ein Schneckenelement können verschiedene Formen verwendet werden.

Zudem sind mehrere Funktionszonen entlang einer axialen Richtung der Schneckenwelle eingestellt; insbesondere sind eine Förderzone, eine Plastifizierungszone, eine weitere Förderzone, eine Knetzone, eine Kühlzone, eine weitere Knetzone und eine weitere Kühlzone in der Reihenfolge vorgesehen; praktisch beginnend mit einem Abschnitt bei der Gummizuführung. Vergleicht man die Förderzone und die Knetzone ist die Steigung der Schneckenflügel in der Knetzone halb so groß wie in der Förderzone. Außerdem sind in einer Knetzone mehrere Kerben in Umfangsrichtung gebildet in Richtung der Schneckenklinge. Außerdem ist in einer Knetzone ein Scherstift vorgesehen, der von der Innenfläche des Zylinders vorsteht. Durch diesen Scherstift wird eine Scherkraft erzeugt, um den Kneteffekt zu verstärken. Die Plastifizierungszone ist wie die Knetzone eingestellt.

In Fig. 4 der JP 2005 001231 A ist eine Konfiguration eines Elements für eine Kühlzone gezeigt mit einem Körper und einem Schneckenblatt des Elements. Die Steigung bzw. der Pitch des Schneckenblatts beträgt 2/3 derjenigen von einem Element der Förderzone. Der Pitch kann auf 2/3 oder weniger eingestellt werden. Dadurch erhöht sich die Verweilzeit des Gummis in der Kühlzone. Somit kann ein Wärmeaustausch mit Kühlwasser innerhalb der Schnecke und innerhalb des Zylinders effizient erfolgen. Ein Eingriffszahnabschnitt ist an der Innenwandfläche eines Durchgangslochs eines Hauptabschnitts des Körpers vorgesehen.

Die Erfindung geht aus von einem Einwellenextruder nach dem Oberbegriff des Anspruchs 1 zum Ändern einer Morphologie von superabsorbierendem Polymergel (SAP-Polymergel), nämlich aufweisend:
- eine Eingangsöffnung zum Zuführen von SAP-Polymergel,
- einen mit der Eingangsöffnung verbundenen Leitungskanal,
- eine im Leitungskanal angeordnete Schneckenwelle zum Fördern und Ändern der Morphologie des SAP-Polymergels und
- eine mit dem Leitungskanal verbundene Ausgangsöffnung, vorzugsweise ebenfalls zum Ändern der Morphologie, zum Abführen des SAP-Polymergels mit geänderter Morphologie,

Die Schneckenwelle weist eine Welle und eine an der Welle angeordnete Schnecke auf und ist angeordnet und ausgebildet das SAP-Polymergel mittels der Schnecke von der Eingangsöffnung zur Ausgangsöffnung zu befördern.

Der Leitungskanal weist eine an oder nahe der Eingangsöffnung angeordnete Eingangszone, eine an oder nahe der Ausgangsöffnung angeordnete Ausgangszone und eine sich entlang des Leitungskanals von der Eingangszone zur Ausgangszone erstreckende Förderzone auf.

Solche Einwellenextruder sind generell aus dem Stand der Technik bekannt und dienen unter anderem dazu feste oder dickflüssige Massen nach dem Funktionsprinzip eines Schneckenförderers zu fördern und der Masse eine Form zu geben.

Solche Einwellenextruder weisen beispielsweise eine in einem Gehäuse angeordnete Schneckenwelle bzw. Schraubenwelle auf, die typischerweise von einem Antriebsmotor um ihre Längsachse rotiert wird. Die Schneckenwelle bzw. Schraubenwelle hat eine Schnecke bzw. Schraube und eine Welle. Die Schnecke bzw. Schraube erstreckt sich entlang einer Längsachse der Welle. Das Gehäuse ist typischerweise zylinderförmig ausgestaltet, so dass die Schnecke der Schneckenwelle an die Gehäusewand angrenzt. Der Innenraum des Gehäuses lässt sich in eine Eingangszone, eine Förderzone und eine Ausgangszone, sog. Dosierungszone einteilen. Masse wird typischerweise über einen Trichter und über eine Eingangsöffnung in die Eingangszone des Einwellenextruders eingeführt. Wenn der Einwellenextruder mit einer Masse befüllt ist, führt die Drehung der Welle der Schneckenwelle zur Förderung der Masse entlang des Gehäuses von der Eingangszone über die Förderzone zur Ausgangszone. Weiterhin wird eine Reibung zwischen der rotierenden Masse und der stehenden Gehäusewand erzeugt, wodurch Druck aufgebaut werden kann und eine Extrusion bzw. ein Herauspressen durch eine formgebende Ausgangsöffnung am Ende der Ausgangszone, d.h. hinter der Dosierungszone, des Einwellenextruders ermöglicht wird.

Eine solche oben genannte Schnecke hat neben anderen Parametern eine Ganghöhe G bzw. Steigung und einen Wellendurchmesser d, sowie einen Schneckenaußendurchmesser D. Die Ganghöhe entspricht dem Abstand zwischen zwei Schneckenwindungen. Über das Verhältnis von Wellendurchmesser d zu Schneckenaußendurchmesser D, d.h. d/D, können Schnecken kategorisiert werden. Bei einem Verhältnis d/D zwischen 0,3 und 0,5 handelt es sich um eine sogenannte tief eingeschnittene Schnecke, die einen hohen Durchsatz ermöglicht. Bei einem Verhältnis d/D zwischen 0,7 und 0,9 handelt es sich um eine sogenannte flach eingeschnittene Schnecke, die einen hohen Druck ermöglicht, aber einen geringeren Durchsatz hat als die tief eingeschnittene Schnecke.

In EP 0 490 361 A1 wird ein Misch-Extruder für schwer mischbare Extrudate beschrieben, bei dem an sich bekannte, und bisher nur einzeln verwendete Stiftzylinder und Transfer-Mischteile gemeinsam in einem Extruder eingesetzt werden - im Transfermischteil im Bereich des größten Gehäusegangvolumens angeordnete Drosselstifte ermöglichen den universellen Einsatz des Extruders in Bezug auf das Extrudat. Der Extruder zur Verarbeitung und Herstellung von Kautschuk und thermoplastischen Kunststoffen besteht aus einem Extrudergehäuse mit einer Einlaß- und einer Auslaßöffnung sowie einem Antrieb für eine im Verarbeitungsraum des Gehäuses um ihre Längsachse drehbar angeordnete Extruderschnecke. Der Extruder verfügt über zwei hintereinander angeordnete Misch- und Homogenisierbereiche. Der eine Bereich ist als Stiftzylinderbereich mit radial in den Verarbeitungsraum des Gehäuses hineinragenden Stiften und in dem Bereich der Stifte unterbrochenen Schneckenstegen ausgebildet. Der andere Bereich ist als Transferbereich ausgelegt, in dem die Extruderschnecke von einem Einlaufbereich zu einem Auslaufbereich eine stetige Verringerung ihres Gangvolumens bis auf Null und dann eine Gangvolumenvergrößerung bis auf einen Maximalwert aufweist. Im Bereich des maximalen Gangvolumens der Gehäusegänge sind radial verstellbare Drosselstifte durch das Gehäuse und in die Gehäusegänge sowie in den Verarbeitungsraum hineinragbar angeordnet. Im Einlauf- und Auslaufbereich des Transferbereichs ist die Gangzahl und somit die Anzahl der Schneckenstege und der Gehäusestege konstant; unabhängig von der Gehäuse- und Schneckengangs-Querschnittsfläche. Im Einzugsbereich des Extruders ist die Gehäuseinnenseite mit einer wendelförmigen Nut versehen.

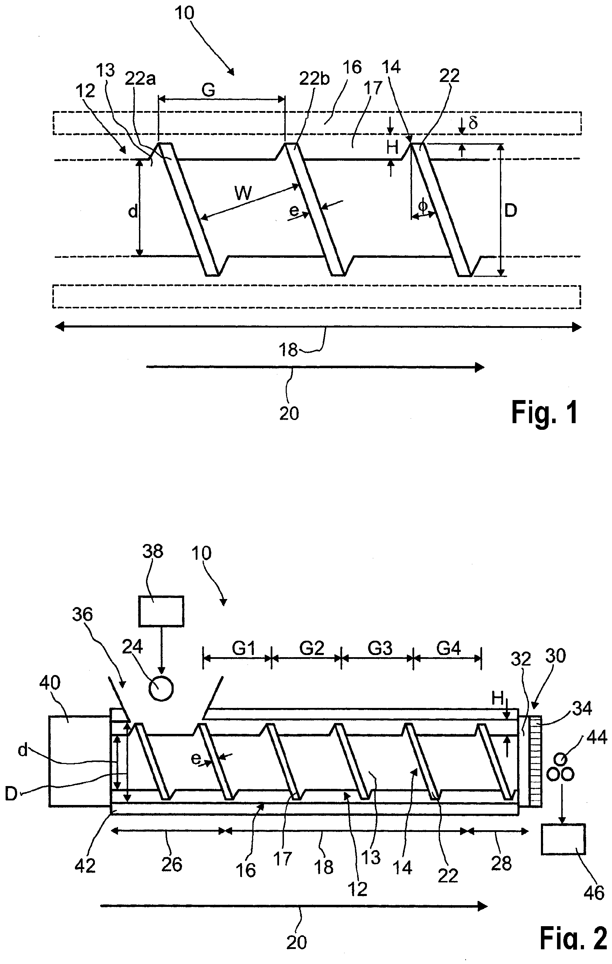

EP 1 510 317 B1 zeigt in ihrer Fig.1 einen Schraubenextruder mit einer nach oben geöffneten Zuführöffnung, einem zylindrischen Rohrteil und einem Schraubenteil. Der Schraubenteil wird durch einen Motor angetrieben und rotiert in dem zylindrischen Rohrteil. Am vorderen Ende des Rohrteils befindet sich eine perforierte Extrusionsplatte mit einer Vielzahl kleiner Löcher. Das Schraubenteil befördert geschnittene Stückchen entlang des Rohrteils zur perforierten Extrusionsplatte. Die Stückchen werden durch die Extrusionsplatte durchgedrückt und zu Hydrogelpartikeln geformt. Die Ganghöhe der Schraube ist gemäß dieser Fig.1 in der Eingangszone größer als in der Förderzone.

EP 2 557 095 A1 zeigt in ihrer Fig.1 einen Schraubenextruder mit einem zylindrischen Gehäuse, einer Basis, einer Schraube, einer Zufuhröffnung, einem Trichter, einer Extrusionsöffnung, einer löchrigen Düse, einer rotierenden Klinge, einem Ring, einem Rückflussverhinderungsteil und einem Motor. Hydrogel wird über den Trichter der Zuführöffnung von oben in das Gehäuse zugeführt. Die Schraube ist in dem Gehäuse angeordnet. Die rotierende Klinge kann an der Schraube angeordnet sein. Der Motor treibt die Schraube an. Die Schraube wird dazu verwendet das Hydrogel entlang des Schraubenextruders zu fördern, um dieses dann aus der Extrusionsöffnung durch die löchrige Düse und die rotierende Klinge herauszuführen. Die Ganghöhe der Schraube ist gemäß dieser Fig.1 in der Eingangszone größer als in der Förderzone.

JP 2005-272653 A zeigt einen Schraubenextruder zum Verarbeiten eines Hydrogels mit u.a. einem Schraubengehäuse, einem Schraubenschaft, einer Füllöffnung, einer Ausgangsöffnung und einer Schraube. Der Schraubenschaft des Schraubenextruders ist derart ausgebildet, dass ein Zurückfließen von Wasser-enthaltendem Gel auf die Zuführseite verhindert wird. Hierfür hat der Schraubenschaft Seitenabschnittslängen entsprechend dem Verhältnis C=(B/A)x100, wobei C in einem Bereich zwischen 100% und 500% liegt. A entspricht der Seitenabschnittslänge zwischen dem proximalen Ende der Schraube und dem distalen Ende der Füllöffnung und B entspricht der Seitenabschnittslänge zwischen dem distalen Ende der Füllöffnung und dem distalen Ende der Schraube.

JP 2002-177807 A zeigt einen Schraubenextruder für die Verarbeitung eines Hydrogels mit einem Gehäuse, einer Füllöffnung, einer Schraube, einer rotierenden Klinge und einer löchrigen Platte. Über die Füllöffnung wird Rohmaterial in das Gehäuse zugeführt. Das Rohmaterial wird entlang des Gehäuses mit Hilfe der Schraube gefördert. Die rotierende Klinge ist am Ende der Schraube, zwischen dieser und der löchrigen Platte angeordnet, wodurch das Rohmaterial geschnitten und durch diese herausgedrückt werden kann.

In dem Buch "Applied Plastics Engineering Handbook: Processing and Materials", von Myer Kutz, 1. Auflage 2011 ist auf den Seiten 227 bis 267 Parameter einer Schrauben- bzw. Schneckengeometrie von Extrudern für die Plastikverarbeitung definiert. Typische Schrauben haben einen konstanten Helixwinkel von 17,7° und eine konstante Ganghöhe. In Fig.15.22 des Buches ist der Aufbau einer Schnecke mit konstantem Helixwinkel und konstanter Ganghöhe gezeigt. Die Schnecke hat einen sich entlang der Längsachse vergrößernden Wellendurchmesser und gleichbleibenden Schneckenaußendurchmesser. Eine andere Schnecke mit dreifach geteilter jeweils konstanter Ganghöhe hat eine jeweils in einer Zone konstante Ganghöhe; nämlich eine konstante Ganghöhe mit einem Helixwinkel von 17,7° in einer Eingangszone, eine größere konstante Ganghöhe in einer Förderzone und eine konstante Ganghöhe mit einem Helixwinkel von 17,7° in der Ausgangszone.

Aufgabe der Erfindung ist es, einen verbesserten Einwellenextruder zum Ändern einer Morphologie von superabsorbierendem Polymer, nämlich eines SAP-Polymergels, zu schaffen bei dem ein möglichst geringer spezifischer mechanischer Energieeinsatz (SME) für einen möglichst hohen Durchsatz aufgewendet werden muss.

Diese Aufgabe wird erfindungsgemäß von einem Einwellenextruder zum Ändern einer Morphologie von superabsorbierendem Polymergel (SAP-Polymergel) gelöst, der eine Eingangsöffnung, einen Leitungskanal, eine Schneckenwelle und eine Ausgangsöffnung aufweist. Die Eingangsöffnung ist zum Zuführen von SAP-Polymergel ausgebildet. Der Leitungskanal ist mit der Eingangsöffnung verbunden. Die Schneckenwelle ist im Leitungskanal angeordnet und zum Fördern und Ändern der Morphologie des SAP-Polymergels ausgebildet. Die Ausgangsöffnung ist mit dem Leitungskanal verbunden, vorzugsweise zum Ändern der Morphologie, zum Abführen des SAP-Polymergels mit geänderter Morphologie ausgebildet. Die Schneckenwelle weist eine Welle und eine an der Welle angeordnete Schnecke auf und ist angeordnet und ausgebildet das SAP-Polymergel mittels der Schnecke von der Eingangsöffnung zur Ausgangsöffnung zu befördern. Der Leitungskanal weist eine an oder nahe der Eingangsöffnung angeordnete Eingangszone, eine an oder nahe der Ausgangsöffnung angeordnete Ausgangszone und eine sich entlang des Leitungskanals von der Eingangszone zur Ausgangszone erstreckende Förderzone auf. Die Schneckenwelle hat einen Ganghöhenwert einer Ganghöhe der Schnecke entlang der Förderzone des Leitungskanals.

Erfindungsgemäß weist der Leitungskanal wenigstens eine Mischelementanordnung mit wenigstens einem Mischelement auf, das in den Leitungskanal des Einwellenextruders hineinragt und das zum Mischen des SAP-Polymergels ausgebildet ist. Vorteilhaft kann der Ganghöhenwert fest sein oder variieren.

Das SAP-Polymergel kann beispielsweise einen Superabsorber aufweisen, wie beispielsweise ein Copolymer aus Arcylsäure und Natriumacrylat. In einem trockenen Zustand liegt das superabsorbierende Polymer (SAP) in Form eines Pulvers vor. Ein trockener Zustand beschreibt hier einen Zustand, in dem das SAP nicht mit Flüssigkeit in Kontakt gekommen ist oder getrocknet wurde. Ist das SAP mit Flüssigkeit in Kontakt gekommen, quillt dieses auf und bildet ein Hydrogel bzw. Polymergel. Der Einwellenextruder ist dazu ausgebildet ein, beispielsweise aus einem Polymerisationsverfahren hervorgegangenes, SAP-Polymergel zu fördern und dessen Morphologie zu ändern. Das SAP-Polymergel liegt also in einem Zustand vor, in dem das SAP mit Flüssigkeit in Kontakt gekommen ist.

Die Erfindung hat erkannt, dass bei hohem Durchsatz von SAP-Polymergel durch aus dem Stand der Technik bekannte Einwellenextruder typischerweise eine unerwünschte Morphologie erzeugt oder eine bevorzugte Morphologie des SAP-Polymergels zerstört werden könnte. Eine erwünschte Morphologie des SAP-Polymergels sieht eine Verbesserung der Wasserabsorption vor, insbesondere indem die Oberflächenporösität und/oder Permeabilität des SAP-Polymergels erhöht wird. Es war bisher schwierig den Durchsatz zu erhöhen ohne die erwünschte Morphologie des SAP-Polymergels zu verlieren. Ein Aspekt der Erfindung ist es, dass ein relativ hoher Durchsatz an SAP-Polymergel durch den Einwellenextruder bei gleichzeitiger Erzeugung von SAP-Polymergel mit einer erwünschten Morphologie möglich ist.

Die Erfindung schließt die Erkenntnis ein, dass es vorteilhaft ist die Geometrie der Schnecke des Einwellenextruders auf das zu befördernde und morphologisch zu verändernde SAP-Polymergel anzupassen, so dass ein möglichst geringer spezifischer mechanischer Energieeinsatz aufgewendet werden muss. Der Einwellenextruder ist also dafür vorgesehen eine erwünschte Morphologie zu erzeugen, welche die Wasserabsorption verbessert, ohne dabei eine große Menge an spezifischer mechanischer Energie (SME) in das SAP-Polymergel einzuführen. Ein weiterer Aspekt der Erfindung ist es, dass die Erfindung einen hohen Durchsatz von SAP-Polymergel ermöglicht. Weiterhin ermöglicht die Erfindung die Herstellung von haltbarem SAP-Polymergel. Insbesondere kann die Erfindung die Beförderungseigenschaften des SAP-Polymergels entlang des Leitungskanals verbessern. Des Weiteren kann eine vorteilhafte Polymermorphologie erzielt werden oder erhalten werden, während der Durchsatz an SAP-Polymergel durch den Leitungskanal erhöht werden kann. Ein weiterer Aspekt ist, dass ein geringerer Energieeinsatz pro Material nötig ist, da eine höhere Beförderungseffizienz möglich ist und konsequenterweise dadurch ein höherer Durchsatz ermöglicht wird. Im Ergebnis kann somit ein kleinerer und kosteneffizienterer Einwellenextruder bereitgestellt werden.

Nachdem die Morphologie mit Hilfe des Einwellenextruders geändert wurde und eine gewünschte Morphologie des SAP-Polymergels vorliegt, kann das SAP-Polymergel beispielsweise einem Trocknungsverfahren zugeführt werden, um das SAP zu trocknen. Bevorzugt soll die Morphologie des SAP-Polymergels derart geändert werden, dass die Wasserabsorption und/oder Absorptionsgeschwindigkeit des SAP-Polymergels erhöht wird. Dies ist beispielweise möglich, indem die Oberflächenporösität und/oder Permeabilität erhöht wird. Insgesamt sollten die wasserabsorbierenden Polymerpartikel eine hohe Quellgeschwindigkeit und hohe Zentrifugenretentionskapazität bei gleichzeitig hoher Permeabilität aufweisen.

Auf das SAP können weitere Verfahrensschritte angewendet werden. Beispielsweise kann das im Trocknungsverfahren getrocknete SAP in einem Mahlverfahren mit Hilfe einer Mühle gemahlen werden, so dass SAP-Partikel mit verschiedenen Größen entstehen. Diese können nachfolgend beispielsweise mit Hilfe von Sieben mit verschiedenen Porenöffnungen ausgesiebt werden, wodurch SAP-Partikel mit verschiedenen Größen getrennt werden können. Die derart getrennten verschieden großen SAP-Partikel können dann beispielsweise nach gewünschten Größenmischungen wieder gemischt werden um eine SAP-Partikel-Mischung mit gewünschten Eigenschaften, wie beispielsweise einer bestimmten Partikelgrößenverteilung, sog. particle size distribution (PSD) zu erhalten. Anschließend können noch weitere Verfahrensschritte, wie beispielsweise eine Oberflächen-Nachvernetzung, sog. surface cross linking (SXL), durchgeführt werden, um die Eigenschaften der SAP-Partikel-Mischung weiter zu verbessern. Bei der SXL werden weitere Stoffe auf die Oberfläche jedes SAP-Partikels aufgebracht. Unter Erhitzung der SAP-Partikel-Mischung mit den auf der Oberfläche befindlichen Stoffen bildet sich ein Netzwerk auf der Oberfläche der jeweiligen Partikel. Dieses Netzwerk kann beispielsweise helfen in einem aufgequollenen Zustand, d.h. wenn die SAP-Partikel-Mischung mit einer Flüssigkeit in Kontakt gekommen ist und die Form eines SAP-Polymergels angenommen hat, die Form der Partikel zu bewahren und so das Haltevermögen bzw. Zentrifugenretentionskapazität bei absorbierter Flüssigkeit zu erhöhen. Insbesondere kann durch das zusätzliche Oberflächennetzwerk das Haltevermögen bei erhöhtem Druck auf das SAP-Polymergel erhöht werden.

Vorteilhafte Weiterbildungen der Erfindung sind den Unteransprüchen zu entnehmen und geben im Einzelnen vorteilhafte Möglichkeiten an, das oben erläuterte Konzept im Rahmen der Aufgabenstellung sowie hinsichtlich weiterer Vorteile zu realisieren.

Vorzugsweise hat die Schneckenwelle einen ersten Ganghöhenwert einer Ganghöhe der Schnecke entlang der Förderzone des Leitungskanals und einen in Beförderungsrichtung folgenden zweiten Ganghöhenwert der Ganghöhe der Schnecke entlang der Förderzone des Leitungskanals. Der zweite Ganghöhenwert ist vorzugsweise größer als der erste Ganghöhenwert.

In einer bevorzugten Weiterbildung weist die Schneckenwelle entlang des Leitungskanals wenigstens drei unterschiedliche Ganghöhenwerte der Schnecke auf. Beispielsweise kann die Schnecke drei verschiedene Ganghöhenwerte haben, von denen der erste Ganghöhenwert der kleinste ist. Der zweite Ganghöhenwert ist in diesem Fall bevorzugt am zweitkleinsten und der dritte Ganghöhenwert ist am größten. Es kann beispielsweise der erste Ganghöhenwert in der Eingangszone liegen und die zwei anderen Ganghöhenwerte in der Förderzone. Es können jedoch beispielsweise auch alle drei unterschiedlichen Ganghöhenwerte in der Förderzone liegen, wobei ein erster Ganghöhenwert in Beförderungsrichtung entlang des Leitungskanals beispielsweise kleiner als ein dritter Ganghöhenwert in Beförderungsrichtung entlang des Leitungskanals, jedoch größer als ein zweiter Ganghöhenwert in Beförderungsrichtung entlang des Leitungskanals sein kann. Die Ganghöhe kann also auch erst abnehmen und danach wieder zunehmen oder zuerst zunehmen und danach wieder abnehmen. Die Schneckenwelle kann beispielsweise auch vier unterschiedliche Ganghöhenwerte entlang des Leitungskanals aufweisen, z.B. zwei unterschiedliche Ganghöhenwerte entlang der Eingangszone und zwei unterschiedliche Ganghöhenwerte entlang der Förderzone, d.h. es kann auch bereits in der Eingangszone ein erster Ganghöhenwert kleiner als ein in Beförderungsrichtung nachfolgender zweiter Ganghöhenwert sein. Die Schneckenwelle kann auch in der Ausgangszone unterschiedliche Ganghöhenwerte aufweisen, insbesondere kann auch in der Ausgangszone ein erster Ganghöhenwert kleiner als ein in Beförderungsrichtung nachfolgender zweiter Ganghöhenwert sein.

In einer Weiterbildung weist die Schneckenwelle eine sich entlang der Förderzone des Leitungskanals wenigstens zweimal ändernde Ganghöhe der Schnecke auf.

In einer weiteren bevorzugten Weiterbildung nimmt die Ganghöhe der Schnecke entlang der Förderzone des Leitungskanals zu, d.h. ein am Übergang zwischen der Eingangszone und Förderzone liegender erster Ganghöhenwert ist kleiner als alle in Beförderungsrichtung folgenden Ganghöhenwerte und der am Übergang zwischen der Förderzone und Ausgangszone liegende Ganghöhenwert ist der größte entlang der Förderzone. Bevorzugt hat die Schnecke also einen kleinsten Ganghöhenwert entlang der Förderzone an einem Übergang von der Eingangszone zur Förderzone und einen größten Ganghöhenwert entlang der Förderzone an einem Übergang von der Förderzone zur Ausgangszone. Besonders bevorzugt ist jeder in Beförderungsrichtung nachfolgende Ganghöhenwert entlang der Förderzone größer oder wenigstens gleich groß wie der in Beförderungsrichtung vorhergehende Ganghöhenwert. Die Ganghöhe in der Förderzone kann entlang der Beförderungsrichtung beispielsweise linear, exponentiell oder stufenweise zunehmen. Dies ermöglicht es entlang der Förderzone das Volumen in dem sich das SAP-Polymergel befindet zu vergrößern.

Dadurch, dass die Ganghöhe der Schnecke entlang der Förderzone des Leitungskanals zunimmt, kann der auf das SAP-Polymergel wirkende Druck entlang der Förderzone zwischen Eingangsöffnung und Ausgangsöffnung verringert werden.

Vorzugsweise nimmt die Ganghöhe der Schnecke entlang der Förderzone des Leitungskanals kontinuierlich zu. Dies ermöglicht eine kontinuierliche Erhöhung des zum Fördern verwendeten Volumens entlang der Förderzone zwischen Eingangsöffnung und Ausgangsöffnung. Zudem ermöglicht dies eine kontinuierliche Druckverringerung entlang der Förderzone zwischen Eingangsöffnung und Ausgangsöffnung. Eine kontinuierliche Druckänderung ermöglicht eine homogenere SAP-Polymergel Herstellung und kann das Förderverhalten verbessern.

Erfindungsgemäß weist der Leitungskanal des Einwellenextruders wenigstens eine Mischelementanordnung mit wenigstens einem Mischelement auf, das in den Leitungskanal des Einwellenextruders hineinragt und das zum Mischen des SAP-Polymergels ausgebildet ist.

Bevorzugt ist das Mischelement innerhalb eines Innenraums des Leitungskanals angeordnet. Die Mischelementanordnung kann sich entlang eines Teils des Leitungskanals oder über die gesamte Länge des Leitungskanals erstrecken oder angeordnet sein. Bevorzugt erstreckt sich die Mischelementanordnung wenigstens entlang der Förderzone oder ist entlang dieser angeordnet. Die Mischelementanordnung ist bevorzugt wenigstens teilweise um den Umfang der Welle der Schneckenwelle herum angeordnet.

Die Mischelementanordnung kann beispielsweise einen oder mehrere in den Innenraum des Leitungskanals hineinragende Stiftanordnungen aufweisen, die entlang der gesamten Länge des Leitungskanals verteilt, beispielsweise an mehreren Stellen entlang der Achse der Schneckenwelle angeordnet sind - eine Stiftanordnung kann Stifte, Bolzen oder dergleichen aufweisen, die umfänglich um die Schneckenwelle verteilt an der Stelle entlang der Achse der Schneckenwelle angeordnet sind. Alternativ oder zusätzlich kann eine Mischelementanordnung eine oder mehrere Aussparungen in der Schnecke der Schneckenwelle aufweisen, die entlang der gesamten Länge des Leitungskanals verteilt, beispielsweise an mehreren Stellen entlang der Achse der Schneckenwelle angeordnet sind. In diese Aussparungen können die Stifte, Bolzen oder dergleichen der Stiftanordnung an der entsprechenden Stelle entlang der Achse der Schneckenwelle eingreifen. Die Stifte, Bolzen oder dergleichen Mischelemente einer Stiftanordnung und entsprechende Aussparungen in der Schneckenwelle dienen der Durchmischung und Erhöhung der Scherung des entlang des Innenraums des Leitungskanals geförderten SAP-Polymergels an der Stelle der Stiftanordnung. Bevorzugt sind die Stifte in einer Wand des Leitungskanals angeordnet, insbesondere befestigt und ragen in den Innenraum des Leitungskanals hinein.

Besonders bevorzugt weist die Mischelementanordnung wenigstens einen sich in den Innenraum des Leitungskanals erstreckenden Stift und wenigstens eine Aussparung entlang der Schnecke auf. Vorzugsweise sind Stift und Aussparung derart angeordnet, dass sich der Stift in die Aussparung der Schnecke erstreckt, wenn die Schnecke im Betrieb rotiert wird, so dass ein Rotieren der Welle der Schneckenwelle möglich ist, ohne dass der Stift die Schnecke berührt. Die Aussparung der Schnecke stellt einen sogenannten cut-out dar, d.h. in diesem Fall ist ein Abschnitt der Schnecke der Schneckenwelle entfernt. Bevorzugt sind mehrere Stifte entlang des Umfangs des Innenraums des Leitungskanals angeordnet, so dass diese in Beförderungsrichtung entlang des Leitungskanals wie ein Gitter wirken. Das durch das von den Stiften gebildete Gitter geführte SAP-Polymergel kann hierdurch gemischt und geschert werden. Die Weiterbildung des Mischelements mit Stiften in Aussparungen der Schnecke ermöglicht es also die Morphologie des SAP-Polymergels im Einwellenextruder zu ändern. Hierbei wird eine erwünschte Morphologie erzeugt, die eine ausreichend hohe Porösität hat, damit die Wasserabsorption verbessert werden kann ("Oberflächenaktivierung"). Die Stifte in den Aussparungen erhöhen insbesondere für größer dimensionierte Einwellenextruder, insbesondere Produktionsversionen des Einwellenextruders, die Durchmischung. Für kleiner dimensionierte Einwellenextruder, insbesondere Laborversionen eines Einwellenextruders ist eine geringere aber deutliche Verbesserung der Durchmischung und eine Erhöhung der Scherung feststellbar.

Erfindungsgemäß weist der Leitungskanal wenigstens zwei entlang des Leitungskanals in der Beförderungsrichtung mit einem Mischelementabstand voneinander getrennt angeordnete Stifte auf. Ein Wert des Mischelementabstands ist erfindungsgemäß auf einen Ganghöhenwert der Ganghöhe der Schnecke angepasst. Es kann der Mischelementabstand derart auf die Ganghöhe angepasst sein, dass ein größerer Wert des Mischelementabstands vorgesehen ist, wenn ein größerer Ganghöhenwert vorgesehen ist und ein kleinerer Wert des Mischelementabstands vorgesehen ist, wenn ein kleinerer Ganghöhenwert vorgesehen ist. Bevorzugt sind mehrere Stifte um den Umfang einer Welle angeordnet, so dass in dem Raum zwischen den jeweils zwei getrennt angeordneten Stiften eine Leitungskanalkammer gebildet wird. Zwischen zwei Schneckenwindungen, d.h. mit der Länge der Ganghöhe, wird eine Schneckenkammer gebildet. Die Länge der Leitungskanalkammer kann auf die Länge der Schneckenkammer angepasst sein. Bevorzugt verhalten sich die Längen gleichläufig, d.h. bei zunehmender Ganghöhe nimmt die Länge der Leitungskanalkammer zu. In einer Weiterbildung nimmt die Länge der Leitungskanalkammer entlang der Förderzone in Beförderungsrichtung zu, beispielsweise linear, exponentiell oder dergleichen.

In einer weiteren Weiterbildung kann der Einwellenextruder eine Schnecke mit wenigstens einem Ganghöhenwert der Schnecke aufweisen, die zwischen 30 mm und 800 mm, beispielsweise zwischen 500 mm und 600 mm liegt. Die Schnecke kann beispielsweise einen ersten Ganghöhewert mit 500 mm, einen zweiten Ganghöhenwert mit 550 mm, und einen dritten Ganghöhenwert mit 600 mm aufweisen. Die Schnecke kann beispielsweise auch lediglich zwei verschiedene Ganghöhenwerte aufweisen, beispielsweise 500 mm und 600 mm. Die Ganghöhenwerte der Schnecke können sich auch kontinuierlich entlang des Leitungskanals zwischen 30 mm und 800 mm, insbesondere zwischen 500 mm und 600 mm verändern. Beispielsweise kann die Ganghöhe sich kontinuierlich entlang des Leitungskanals ändern, indem sich der Ganghöhenwert von 500 mm nahe der Eingangsöffnung auf 600 mm nahe der Ausgangsöffnung erhöht.

Vorzugsweise hat der Einwellenextruder einen Wert einer Laufbreite der Schnecke zwischen 4 mm und 80 mm, beispielsweise 40 mm.

In einer Weiterbildung kann der Einwellenextruder wenigstens einen Wert einer Förderraumhöhe zwischen der Welle der Schneckenwelle und einer Wand des Leitungskanals zwischen 25 mm und 500 mm, beispielsweise 310 mm haben.

Vorzugsweise hat der Einwellenextruder einen Wert eines Schneckenaußendurchmessers zwischen 60 mm und 1000 mm, beispielsweise 650 mm. Bevorzugt hat der Einwellenextruder wenigstens einen Wert eines Wellendurchmessers zwischen 25 mm und 500 mm, beispielsweise 340 mm.

Der Einwellenextruder hat vorzugsweise ein Verhältnis zwischen Wellendurchmesser zu Schneckenaußendurchmesser zwischen 0,4 und 0,6, beispielsweise zwischen 0,449 und 0,577. Besonderes bevorzugt hat der Einwellenextruder ein Verhältnis zwischen Wellendurchmesser zu Schneckenaußendurchmesser von 0,52. Der Einwellenextruder kann auch ein Verhältnis zwischen Wellendurchmesser zu Schneckenaußendurchmesser zwischen 0,3 bis 0,9 haben. Das Verhältnis des Wellendurchmessers zu Schneckenaußendurchmesser bestimmt das Verhältnis von Durchsatz und auf das SAP-Polymergel wirkenden Druck. Ein größeres Verhältnis von Wellendurchmesser zu Schneckenaußendurchmesser bewirkt einen höheren Druck und einen geringeren Durchsatz, da das Transportvolumen verringert wird. Wenn die Schneckenwelle im Betrieb ist, bilden sich durch die Ganghöhe zwischen den Schneckenwindungen definierte Schneckenkammern mit sich entlang des Leitungskanals vergrößernden freien Transportvolumina. Die sich vergrößernden freien Transportvolumina sind eine Folge der in Beförderungsrichtung zunehmenden Ganghöhenwerte entlang der Förderzone.

In einer bevorzugten Weiterbildung nimmt die Ganghöhe entlang der Förderzone von einem Ganghöhenwert von 500 mm nahe der Eingangszone auf einen Ganghöhenwert von 600 mm nahe der Ausgangszone zu. Durch die zunehmende Ganghöhe kann das freie Transportvolumen der Schneckenkammer erhöht werden, wobei gleichzeitig der auf das SAP-Polymergel wirkende Druck verringert wird.

In einer weiteren bevorzugten Weiterbildung hat die Schneckenwelle einen niedrigsten Ganghöhenwert entlang des Leitungskanals, der höchstens zwischen 20% und 85% eines höchsten Ganghöhenwertes entlang des Leitungskanals beträgt. Die Schneckenwelle kann beispielsweise einen Ganghöhenwert nahe der Eingangszone haben, der höchstens zwischen 20% und 85% eines Ganghöhenwertes nahe der Ausgangszone beträgt. Die Begrenzung der Änderung der Ganghöhe entlang des Leitungskanals verringert das Risiko, dass eine unerwünschte morphologische Änderung des SAP-Polymergels auftritt.

Bevorzugt ist der Einwellenextruder ausgebildet ein SAP-Polymergel mit einer erwünschten die Wasserabsorption des SAP erhöhenden Morphologie und mit einem Durchsatz von wenigstens 23 Tonnen pro Stunde zu erzeugen. Der Einwellenextruder ist hierfür entsprechend als eine Produktionsversion dimensioniert, d.h., dass unter anderem der Leitungskanal und die Schneckenwelle eine entsprechende Größe haben.

In einer besonders bevorzugten Weiterbildung weist der Einwellenextruder einen Antriebsmotor und/oder eine Heizvorrichtung auf. Bevorzugt ist der Antriebsmotor ausgebildet die Welle der Schneckenwelle mit bis zu 150 Umdrehungen pro Minute, beispielsweise mit 75 Umdrehungen pro Minute und bevorzugt mit 30 Umdrehungen pro Minute zu rotieren.

Die Heizvorrichtung ist bevorzugt ausgebildet den Leitungskanal zu erwärmen, so dass dieser eine Temperatur zwischen 85° C und 140° C jedenfalls in der Eingangszone hat. Die Heizvorrichtung kann sich beispielsweise entlang der Länge des Leitungskanals erstrecken und diesen umschließen, so dass eine gleichmäßige Erwärmung möglich ist. Alternativ kann die Heizvorrichtung sich auch nur über Teile des Leitungskanals erstrecken. Die Heizvorrichtung kann auch derart angeordnet und ausgebildet sein, dass sie verschiedene Temperaturzonen erzeugen kann.

Vorzugsweise ist die Heizvorrichtung derart angeordnet und ausgebildet, dass sich beim Betrieb des Einwellenextruder die Temperatur des SAP-Polymergel im Leitungskanal entlang der Beförderungsrichtung bis zu einer Ausgangszone erhöht, vorzugsweise um eine Temperatur im Bereich von 20° C bis 40° C erhöht. Die Temperatur nimmt mit Energieeintrag, insbesondere auch per Erhöhung von Druck und Scherkräften entlang der Beförderungsrichtung zu. Das SAP-Polymergel hat vorzugsweise weiter direkt nach der Ausgangsöffnung eine wieder verringerte Temperatur des wieder abgekühlten SAP-Polymergel zwischen 90° C und 110° C.

Bevorzugt weist die Heizvorrichtung ein Öl mit einer Temperatur von bis zu 150° C auf. Die Heizvorrichtung kann auch zum Kühlen ausgebildet sein und dafür beispielsweise ein Öl mit einer Temperatur aufweisen, das geringer als die Temperatur des SAP-Polymergels ist.

In einer Weiterbildung hat der Leitungskanal eine Länge zwischen 200 mm und 4000 mm. Bevorzugt hat der Leitungskanal eine Länge von wenigstens 3000 mm, so dass die Schnecke eine Arbeitslänge von 3000 mm hat. Der Leitungskanal kann beispielsweise einen Innendurchmesser zwischen 50 mm und 750 mm haben. Bevorzugt hat der Leitungskanal einen Innendurchmesser von 650 mm.

In einer bevorzugten Weiterbildung weist die Ausgangsöffnung eine Düse auf, vorzugsweise ebenfalls zum Ändern der Morphologie, zum Abführen des SAP-Polymergels mit geänderter Morphologie. Die Düse kann eine Durchtrittsöffnung bzw. ein Loch oder mehrere Durchtrittsöffnungen bzw. Löcher haben bzw. es kann diese grundsätzlich eine Anzahl von Durchtrittsöffnungen oder Löcher im Bereich zwischen 60 und 90 haben - in diesem Fall handelt es sich um eine bevorzugte Anzahl von Löchern für die Laborversion des Einwellenextruders. Bevorzugt hat die Düse bei einer Produktionsversion sehr viel mehr Löcher. Besonders bevorzugt hat die Düse mehr als 1000 Löcher, beispielsweise 3600 Löcher. Bei der Produktionsversion des Einwellenextruders hat die Düse bevorzugt eine Anzahl von Löchern im Bereich zwischen 3000 und 4000 Löchern.

Die Löcher können beispielsweise Durchmesser zwischen 4 mm und 12 mm haben. Bevorzugt haben die Löcher einen Durchmesser von 8 mm. Vorzugsweise sind 10% der Fläche der Düse offen, d.h. 10 % der Fläche sind Flächen von Durchtrittsöffnungen bzw. Löchern.

In einer bevorzugten Weiterbildung weist die Ausgangsöffnung eine Düse mit 3000 Löchern auf, die jeweils einen Durchmesser von 8 mm haben. In diesem Fall sind besonders bevorzugt 10% der Fläche der Düse offen.

In einer bevorzugten Weiterbildung ist der Einwellenextruder dazu ausgebildet einen maximalen Druck von 50 bar zu erzeugen. Der Einwellenextruder kann dazu ausgebildet sein eine Durchsatzleistung von 30t/h (Tonnen pro Stunde) zu erzeugen. Der Einwellenextruder kann ferner dazu ausgebildet sein eine spezifische mechanische Energie von bis zu 60 kWh pro Tonne aufzunehmen. Des Weiteren kann der Einwellenextruder dazu ausgebildet sein mit einer Eingangsleistung von bis zu 18,5 kW betrieben zu werden.

Die Erfindung betrifft des Weiteren ein Verfahren zum Ändern der Morphologie eines superabsorbierenden Polymergels (SAP-Polymergels) mit dem Einwellenextruder der Erfindung oder einer der Weiterbildungen. Das Verfahren weist die Schritte auf:
- Zuführen des SAP-Polymergels von einem Polymerisationsverfahrens in den Einwellenextruder,
- Betreiben des Einwellenextruders, um die Morphologie des SAP-Polymergels zu ändern,
- Abführen des SAP-Polymergels mit geänderter Morphologie aus dem Einwellenextruder zu einem Trocknungsverfahren.

In einer Weiterbildung des Verfahrens umfasst das Trocknungsverfahren die Schritte:
- Verteilen des SAP-Polymergels mit geänderter Morphologie auf Trocknungsblechen und
- Erhitzen des SAP-Polymergels mit geänderter Morphologie auf den Trocknungsblechen in einem Konvektionsofen bzw. Umluftofen.

Die Trocknungsbleche können mit verschiedenen Mengen an SAP-Polymergel mit geänderter Morphologie befüllt werden, so dass sich ein Befüllungsgrad in Abhängigkeit der Fläche des Trocknungsblechs und der Menge des auf das Trocknungsblech befüllten SAP-Polymergels mit geänderter Morphologie ergibt. Die Trocknungstemperatur und Trocknungsdauer hängen von dem Befüllungsgrad ab, beispielsweise kann bei einer Befüllung mit 0,91 g/cm² eine Trocknungstemperatur von 175°C für 70 Minuten zum Trocknen verwendet werden. Die Trocknungsbleche können einen Siebboden haben, beispielsweise mit 250 µm Maschenöffnungen, so dass die Flüssigkeit auch durch den Siebboden entweichen kann. Der Siebboden kann beispielsweise aus Drähten mit einem Durchmesser von jeweils 130 µm gebildet sein.

In einer Weiterbildung weist das Verfahren den zusätzlichen Schritt auf:
- Zuführen des getrockneten SAP zu einem Mahlverfahren.

Vorzugsweise wird das getrocknete SAP im Mahlverfahren von einer Mühle gemahlen, so dass SAP-Partikel mit unterschiedlichen Größen entstehen.

Das Verfahren kann des Weiteren den folgenden Schritt aufweisen:
- Zuführen des gemahlenen SAP zu einem Siebverfahren bzw. Sortierverfahren.

Vorzugsweise wird das gemahlene SAP im Siebverfahren bzw. Sortierverfahren gesiebt bzw. sortiert, so dass SAP-Partikel mit verschiedenen Größen voneinander getrennt werden.

Das Verfahren kann weiterhin den folgenden Schritt aufweisen:
- Zuführen von sortieren SAP-Partikeln zu einem Mischverfahren.

Vorzugsweise werden im Mischverfahren SAP-Partikel verschiedener Größen entsprechend einem vorbestimmten Mischverhältnis gemischt, um gewünschte Eigenschaften der SAP-Partikel-Mischung zu erhalten.

Des Weiteren kann das Verfahren den folgenden Schritt aufweisen:
- Zuführen der SAP-Partikel-Mischung zu einem Oberflächen-Nachvernetzungsverfahren bzw. surface cross linking (SXL)-Verfahren.

Vorzugsweise wird auf die Oberfläche der SAP-Partikel ein Oberflächenvernetzungsstoff, der beispielsweise Isopropanol, Wasser und/oder Aluminiumlactat enthalten kann, aufgebracht. Die mit Oberflächenvernetzungsstoff benetzten SAP-Partikel werden dann bevorzugt bei einer Temperatur von 185° C erwärmt, so dass sich eine Oberflächenvernetzung auf den jeweiligen SAP-Partikeln ausbildet. Die Oberflächenvernetzung ermöglicht es den SAP-Partikeln im aufgequollenen Zustand, d.h. wenn die SAP-Partikel Flüssigkeit aufgenommen haben und als Polymergel vorliegen ihre Form zu behalten. Insbesondere kann durch die Oberflächenvernetzung das Haltevermögen bei absorbierter Flüssigkeit unter Druck gesteigert werden.

Die Erfindung betrifft weiterhin eine Verwendung des Einwellenextruders der Erfindung oder einer der Weiterbildungen zum Ändern einer Morphologie von superabsorbierendem Polymergel (SAP-Polymergel).

Ausführungsbeispiele der Erfindung werden nun nachfolgend anhand der Zeichnung beschrieben. Diese soll die Ausführungsbeispiele nicht notwendigerweise maßstäblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf den einschlägigen Stand der Technik verwiesen. Dabei ist zu berücksichtigen, dass vielfältige Modifikationen und Änderungen betreffend die Form und das Detail einem Ausführungsbeispiel vorgenommen werden können, ohne von der allgemeinen Idee der Erfindung abzuweichen. Die in der Beschreibung, in der Zeichnung sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, der Zeichnung und/oder den Ansprüchen offenbarten Merkmale. Die allgemeine Idee der Erfindung ist nicht beschränkt auf die exakte Form oder das Detail der im folgenden gezeigten und beschriebenen bevorzugten Ausführungsbeispiele oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsbeispiele sowie anhand der Zeichnung; diese zeigt in:
- Fig.1: eine schematische Darstellung eines Ausschnitts eines beispielhaften Einwellenextruders mit einer Schneckenwelle mit Parameterdefinitionen gemäß einer Ausführungsform der Erfindung;
- Fig.2: eine schematische Darstellung einer Ausführungsform eines Einwellenextruders in einem bevorzugten Ausführungsbeispiel mit hier nicht gezeigten Mischelementen einer Mischelementanordnung;
- Fig.3: eine schematische Darstellung einer bevorzugten Ausführungsform eines Einwellenextruders gemäß dem Konzept der Erfindung mit Mischelementen einer Mischelementanordnung;
- Fig.4: in Ansichten (A1, A2), (B1, B2) und (C) bevorzugte Varianten von Stiftanordnungen mit Stiften für eine in Fig.3 gezeigte Mischelementanordnung oder für eine Mischelementanordnung in der Ausführungsform der Fig.2;
- Fig.5: ein Ausführungsbeispiel eines ersten bevorzugten Verfahrens zum Ändern der Morphologie eines superabsorbierenden Polymergels (SAPPolymergels) mit einem Einwellenextruder gemäß einer bevorzugten Ausführungsform der Fig.2 (mit dort der Übersichtlichkeit wegen nicht gezeigten Mischelementen, die aber beispielsweise gemäß einer der Varianten der Fig.4 vorgesehen sind) oder Fig.3 (mit den dort gezeigten Mischelementen, die ebenfalls gemäß einer der Varianten der Fig.4 vorgesehen sind);
- Fig.6: ein weiteres Ausführungsbeispiel eines bevorzugten Verfahrens zum Ändern einer Morphologie eines superabsorbierenden Polymergels (SAP-Polymergels) mit einem Einwellenextruder gemäß einer bevorzugten Ausführungsform der Fig. 2 (mit dort der Übersichtlichkeit wegen nicht gezeigten Mischelementen, die aber beispielsweise gemäß einer der Varianten der Fig.4 vorgesehen sind) oder Fig.3 (mit den dort gezeigten Mischelementen, die ebenfalls gemäß einer der Varianten der Fig.4 vorgesehen sind).

Fig.1 zeigt einen Ausschnitt eines Einwellenextruders 10 mit einer Schneckenwelle 12. Die Schneckenwelle 12 weist eine Welle 13, sowie eine sich entlang der Welle 13 erstreckende Schnecke 14 auf und ist in einem Innenraum 17 eines Leitungskanals 16 angeordnet. Der in Fig.1 gezeigte Ausschnitt zeigt einen in einer Förderzone 18 angeordneten Abschnitt der Schneckenwelle 12. Die Beförderungsrichtung 20 entlang der Förderzone 18 verläuft in dieser Darstellung von links nach rechts.

Im Betrieb der Schneckenwelle 12 wird die Welle 13 um ihre Längsachse rotiert, so dass auch die Schnecke 14 rotiert wird und die Schneckenwindungen 22 Material, wie beispielsweise SAP-Polymergel 24 (siehe Fig.2) entlang des Leitungskanals 16 in Beförderungsrichtung 20 fördern. Hierbei wird das Material, beispielsweise SAP-Polymergel 24, einerseits von der Schnecke 14 und der Wand des Leitungskanals 16 geschoben, andererseits jedoch auch gezogen, so dass sich ein komplexes Fließverhalten entwickelt. Die Förderzone 18 fungiert somit als Pumpe, die Material, wie beispielsweise SAP-Polymergel 24, von einer Eingangszone 26 zu einer Ausgangszone 28 befördert (siehe Fig.2). In der Ausgangszone 28 ist ein Druckwert vorteilhaft, der hoch genug ist, um das SAP-Polymergel 24 aus der Ausgangsöffnung 30 durch eine Lochscheibe 32 und eine Düse 34 herauszudrücken (siehe Fig.2); d.h. vorzugsweise zum Ändern der Morphologie des SAP-Polymergels 24. Die Höhe des vorteilhaften Druckwerts hängt unter anderem von der Materialmenge, den viskoseelastischen Eigenschaften des Materials, beispielsweise des SAP-Polymergels 24, und dessen Temperatur ab.

In Abhängigkeit der Parameter der Schneckenwelle 12, insbesondere von Welle 13 und Schnecke 14 kann der Druck und ein Durchsatz des Materials, beispielsweise SAP-Polymergel 24 eingestellt werden. Weiterhin hängen der Durchsatz und Druck auch u.a. von der Umdrehungsgeschwindigkeit der Welle 13 der Schneckenwelle 12 ab. Für die Änderung der Morphologie des SAP-Polymergels 24 werden bestimmte vorteilhafte Drücke benötigt. Daher kann es notwendig sein, die Parameter der Schneckenwelle 12, insbesondere von Welle 13 und Schnecke 14, derart einzustellen, dass ein genügend hoher Druck erreicht wird.

Parameter des Einwellenextruders 10 sind der Wellendurchmesser d (engl.: root diameter), der Schneckenaußendurchmesser D (engl.: diameter), die Ganghöhe G (engl.: lead bzw. pitch), Gangweite W (engl.: channel width), Laufbreite e (engl.: flight width), Förderraumhöhe H (engl.: metering depth), Helixwinkel φ (engl.: helix angle) und der Abstand δ zur Wand des Leitungskanals 16 (engl.: flight clearance). Im Folgenden werden diese Parameter näher erläutert.

Die Welle 13 der Schneckenwelle 12 hat den Wellendurchmesser d. Die Schnecke 14 hat den Schneckenaußendurchmesser D. Die Schnecke 14 der Schneckenwelle 12 kann in Abhängigkeit des Verhältnisses zwischen Wellendurchmesser d zu Schneckenaußendurchmesser D, d.h. d/D, kategorisiert werden. Bei einem Wert von d/D zwischen 0,3 und 0,5 handelt es sich um eine sogenannte tief eingeschnittene Schnecke, die einen hohen Durchsatz ermöglicht. Bei einem Wert von d/D zwischen 0,7 und 0,9 handelt es sich um eine sogenannte flach eingeschnittene Schnecke, die einen hohen Druck ermöglicht, aber einen geringeren Durchsatz hat als die tief eingeschnittene Schnecke. Der Durchsatz der Schnecke 14, also insbesondere auch einer flach eingeschnittenen Schnecke, lässt sich erhöhen, indem die Umdrehungsgeschwindigkeit der Welle 13 der Schneckenwelle 12 erhöht wird. So kann für eine flach eingeschnittene Schnecke ein hoher Druck bei relativ hohem Durchsatz erreicht werden. Allerdings ist hierfür ein relativ hoher Energieeinsatz notwendig, um die hierfür notwendigen hohen Umdrehungszahlen der Welle 13 der Schneckenwelle 12 bereitzustellen.

Die Ganghöhe G der Schnecke 14 gibt den Abstand zwischen dem proximalen Ende einer ersten Schneckenwindung 22a und dem proximalen Ende einer zweiten Schneckenwindung 22b der Schnecke 14 an. Die Gangweite W gibt den Abstand zwischen dem distalen Ende einer ersten Schneckenwindung 22a und dem proximalen Ende einer zweiten Schneckenwindung 22b der Schnecke 14 an. Die Laufbreite e gibt die Breite der Schnecke 14 an. Die Förderraumhöhe H gibt den Abstand zwischen Wand des Leitungskanals 16 und Oberfläche der Welle 13 an. Der Helixwinkel φ gibt den Winkel an, mit dem die Schnecke 14 geneigt ist. Der Abstand δ zur Wand des Leitungskanals 16 gibt den Abstand zwischen der Außenoberfläche der Schnecke 14 und der Wand des Leitungskanals 16 an. Es sollte ein minimaler Abstand δ zur Wand des Leitungskanals 16 eingehalten werden, da sich die Schnecke 14 ansonsten im Betrieb in die Wand des Leitungskanals 16 eindrehen würde und diese beschädigen könnte. Der Abstand δ zur Wand des Leitungskanals 16 ist jedoch im Vergleich zu den sonstigen Dimensionen sehr gering, beispielsweise unter 1 mm.

Fig.2 zeigt ein bevorzugtes Ausführungsbeispiel eines Einwellenextruders 10. Dieser kann verschieden dimensioniert werden, beispielsweise als eine große Produktionsversion des Einwellenextruders 10 die für die industrielle Produktion geeignet ist und als eine kleine Laborversion des Einwellenextruders 10, die zu Versuchszwecken dient. Der Aufbau der beiden Versionen des Einwellenextruders 10 ist identisch. Die Einwellenextruder 10 unterscheiden sich vor allem in der Dimensionierung. Insbesondere ist die große Produktionsversion des Einwellenextruders 10 für einen Durchsatz von bis zu 30 Tonnen pro Stunde ausgebildet, während die Laborversion des Einwellenextruders 10 lediglich für einen Durchsatz von bis zu 340kg/h (Kilogramm pro Stunde) ausgebildet ist. Die Laborversion des Ausführungsbeispiels des Einwellenextruders 10 ist eine herunterskalierte Version der Produktionsversion des Ausführungsbeispiels des Einwellenextruders 10 und kann insbesondere für Versuchszwecke verwendet werden, beispielsweise um Vergleichsmessungen für verschiedene Schneckenwellen 12 durchzuführen.

Der Einwellenextruder 10 hat eine Eingangsöffnung 36, einen Leitungskanal 16, eine Schneckenwelle 12 und eine Ausgangsöffnung 30. Der Leitungskanal 16 hat einen Innenraum 17, dem eine Eingangszone 26, eine Förderzone 18 und eine Ausgangszone 28 zugeordnet ist und sich zwischen der Eingangsöffnung 36 und der Ausgangsöffnung 30 erstreckt. Der Einwellenextruder 10 kann verwendet werden, ein superabsorbierendes Polymergel (SAP-Polymergel 24) in Beförderungsrichtung 20 zu fördern und eine Morphologie des SAP-Polymergels 24 zu ändern. Insbesondere kann der Einwellenextruder 10 dafür verwendet werden, die Oberflächenporösität der Partikel des SAP-Polymergels zu erhöhen, um die Wasserabsorptionsfähigkeit des SAP-Polymergels zu erhöhen.

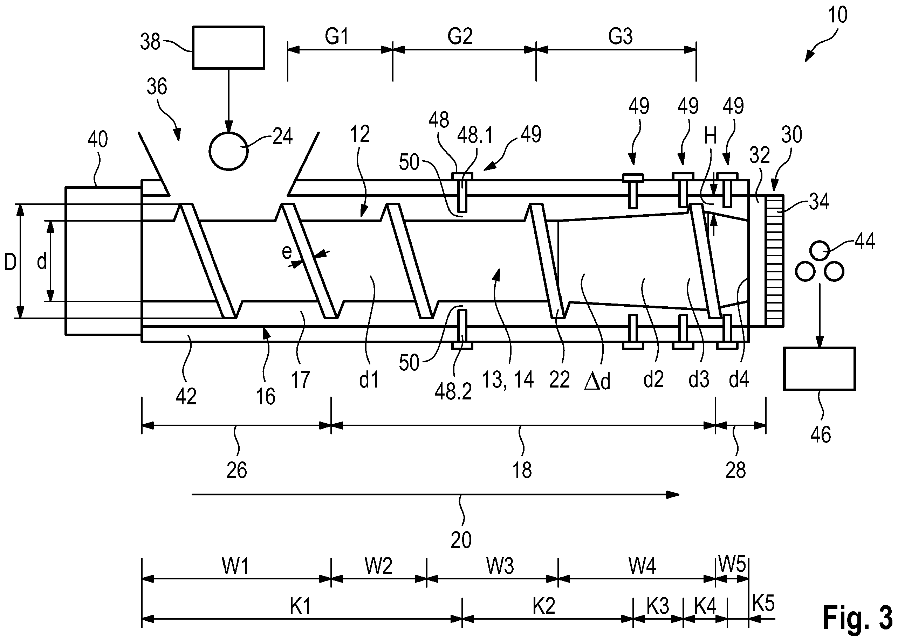

In einem bevorzugten Ausführungsbeispiel des Einwellenextruders 10 in Fig.2, weist dieser Einwellenextruder 10 gemäß dem Konzept der Erfindung ein hier nicht gezeigtes Mischelement auf. Das Mischelement kann --wie in Fig.3 und mit gleicher Funktion und Vorteil-- Stifte im Leitungskanal 16 aufweisen und Aussparungen in der Schnecke 14 der Schneckenwelle 12 aufweisen.

Das SAP-Polymergel 24 wird in einem Polymerisationsverfahren 38 hergestellt. Das sogenannte Basispolymer, auf dem das SAP-Polymergel 24 basiert, wird für das in Fig.2 gezeigte Ausführungsbeispiel entsprechend der in Tabelle 1 gegebenen Formulierung hergestellt.

**Tabelle 1:**

| | |
|---|---|
| Reaktor | ORP 250 |
| feste Bestandteile | 42,7 Gewichts-% |
| Grad der Neutralisierung | 71 mol-% |
| Basispolymerfeindosierung | 7,7 Gewichts-% baP (basierend auf Polymer) |
| Wasserstoffperoxid | 0,0008 Gewichts-% baA (basierend auf Acrylsäure) |
| Ascorbinsäure | 0,0023 Gewichts-% baA |
| Natriumpersulfat | 0,128 Gewichts-% baA |
| Kernvernetzer | 0,39 Gewichts-% baA (Laromer PO9044V) |
| Monomer-Temperatur | 35°C |
| Durchsatz | 800 kg/h |
| Dammhöhe | 160 mm |

Der Einwellenextruder 10 der Fig.2 ist nicht auf die Verarbeitung von SAP-Polymergel 24 mit der Zusammensetzung der Tabelle 1 beschränkt und kann auch für andere Zusammensetzungen eines SAP-Polymergels 24 verwendet werden.

Das im Polymerisationsverfahren 38 hergestellte SAP-Polymergel 24 wird dem Einwellenextruder 10 über die Eingangsöffnung 36 zugeführt. Die Eingangsöffnung 36 ist mit dem Innenraum 17 des Leitungskanals 16 verbunden und grenzt an die Eingangszone 26 an. Im Betrieb des Einwellenextruders 10 wird das SAP-Polymergel 24 über die Eingangsöffnung 36 der Eingangszone 26 des Innenraums 17 des Leitungskanals 16 zugeführt.

Im Innenraum 17 des Leitungskanals 16 ist die Schneckenwelle 12 angeordnet, die eine Welle 13 mit einer Schnecke 14 aufweist. Wenn der Einwellenextruder 10 betrieben wird, wird die Welle 13 der Schneckenwelle 12 um ihre Längsachse rotiert. Hierfür ist ein Antriebsmotor 40 vorgesehen, der mit der Welle 13 der Schneckenwelle 12 verbunden ist. Der Antriebsmotor 40 kann in diesem Ausführungsbeispiel die Welle 13 der Schneckenwelle 12 mit bis zu 150 Umdrehungen pro Minute rotieren. Im Betrieb wird die Welle 13 je nach Abmessungen des Einwellenextruders 10 bevorzugt mit einer Rotationsgeschwindigkeit zwischen 30 Umdrehungen pro Minute für die Produktionsversion des Einwellenextruders 10 und 75 Umdrehungen pro Minute für die Laborversion des Einwellenextruders 10 rotiert. Die Schneckenwelle 12 dient dazu mit Hilfe der Schnecke 14 und der Wand des Leitungskanals 16 das SAP-Polymergel 24 von der Eingangsöffnung 36 entlang der Förderzone 18 zur Ausgangsöffnung 30 zu fördern und die Morphologie des SAP-Polymergels 24 zu ändern.

In diesem Ausführungsbeispiel ist der Leitungskanal 16 des Einwellenextruders 10 vollständig von einer Heizvorrichtung 42 umschlossen. Alternativ kann auch nur ein Teil des Leitungskanals 16 von der Heizvorrichtung 42 umschlossen sein oder gar keine Heizvorrichtung 42 vorhanden sein (nicht gezeigt). Bevorzugt ist die Laborversion des Einwellenextruders 10 mit einer Heizvorrichtung 42 umschlossen, während die Produktionsversion des Einwellenextruders 10 bevorzugt ohne Heizvorrichtung 42 ausgeführt ist. Die Heizvorrichtung 42 dient dazu den Leitungskanal 16 zu erwärmen. Hierfür ist in der Heizvorrichtung 42 ein Öl angeordnet, dass eine Temperatur von bis zu 200°C haben kann.

Die Heizvorrichtung 42 wird bevorzugt derart betrieben, dass die Temperatur eines SAP-Polymergels 24 im Leitungskanal 16, jedenfalls in der Eingangszone 26, zwischen 85°C und 100°C in der Laborversion des Einwellenextruders 10 beträgt und/oder in der Produktionsversion etwas höher liegt. In der Produktionsversion des Einwellenextruders 10 beträgt die Temperatur im Leitungskanal 16 jedenfalls in dem Eingangsbereich bevorzugt zwischen 90°C und 140°C. Die Temperatur nimmt mit Energieeintrag, insbesondere per Erhöhung von Druck und Scherkräften entlang der Beförderungsrichtung 20, zu. Diese Temperaturerhöhung entlang der Beförderungsrichtung 20 bis zu einer Ausgangszone 28 kann zwischen 20°C und 40°C im Vergleich zur Eingangszone 26 betragen.

Vorzugsweise hat das SAP-Polymergel 24 direkt nach der Ausgangsöffnung 30 eine in Folge von Abkühlung wieder geringere Temperatur zwischen 78°C und 110°C, vorzugsweise zwischen 90°C und 110°C.

Die Heizvorrichtung 42 kann auch zum Kühlen verwendet werden, beispielsweise wenn sie mit einem Öl befüllt wird, dessen Temperatur geringer ist als die Temperatur des Leitungskanals 16 und/oder des SAP-Polymergels 24. Die Heizvorrichtung 42 kann grundsätzlich auch dem Zweck einer Temperaturregelung dienen.

Die Ausgangsöffnung 30 ist mit dem Innenraum 17 des Leitungskanals 16 verbunden und grenzt an die Ausgangszone 28 an. Die Ausgangsöffnung 30 weist eine Lochscheibe 32 und eine an die Lochscheibe 32 nachfolgend angeordnete Düse 34 auf. Die Ausgangsöffnung 30 dient dazu die Morphologie des SAP-Polymergels 24 zu ändern und das SAP-Polymergel 44 mit geänderter Morphologie abzuführen. Hierfür wird mit Hilfe des Förderungsmechanismus der Schnecke 14 und Wand des Leitungskanals 16 SAP-Polymergel 24 mit Druck durch die Lochscheibe 32 und durch die Düse 34 gedrückt.

Die Düse 34 hat in diesem Ausführungsbeispiel 66 Durchtrittsöffnungen bzw. es kann diese grundsätzlich eine Anzahl von Durchtrittsöffnungen im Bereich zwischen 60 und 90 haben - in diesem Fall handelt es sich um eine bevorzugte Anzahl von Löchern für die Laborversion des Einwellenextruders 10. Die Düse 34 kann auch nur eine Durchtrittsöffnung bzw. ein Loch haben oder eine andere Anzahl von Löchern. Beispielsweise, jedenfalls für die Produktionsversion eines Einwellenextruders, wird eine bevorzugte Anzahl von Löchern mindestens 1000 beispielsweise 3600 betragen. Bei der Produktionsversion des Einwellenextruders 10 hat die Düse 34 eine Anzahl von Löchern im Bereich zwischen 3000 und 4000 Löchern.

Die Löcher haben einen Durchmesser von jeweils 8 mm. Alternativ können die Löcher beispielsweise auch einen Durchmesser zwischen 4 mm und 12 mm haben. Es können Löcher mit gleich großen Durchmessern oder verschieden großen Durchmessern nebeneinander angeordnet sein. In diesem Ausführungsbeispiel sind für die Laborversion des Einwellenextruders 10 ungefähr 66% der Fläche der Düse 34 offen. Alternativ können auch beispielsweise zwischen 5% und 66% der Fläche der Düse 34 offen sein (nicht gezeigt). In der Produktionsversion des Einwellenextruders 10 sind 10% der Fläche der Düse offen (nicht gezeigt).

Der Einwellenextruder 10 kann zusätzlich oder alternativ neben der Lochscheibe 32 und der Düse 34 an der Ausgangsöffnung 30 einen Adapter, einen Halter, eine Matrix oder eine Kombination dieser Komponenten aufweisen (nicht gezeigt). Eine oder mehrere der vorgenannten Komponenten können beispielsweise am distalen Ende der Schneckenwellen 12 angeordnet sein.

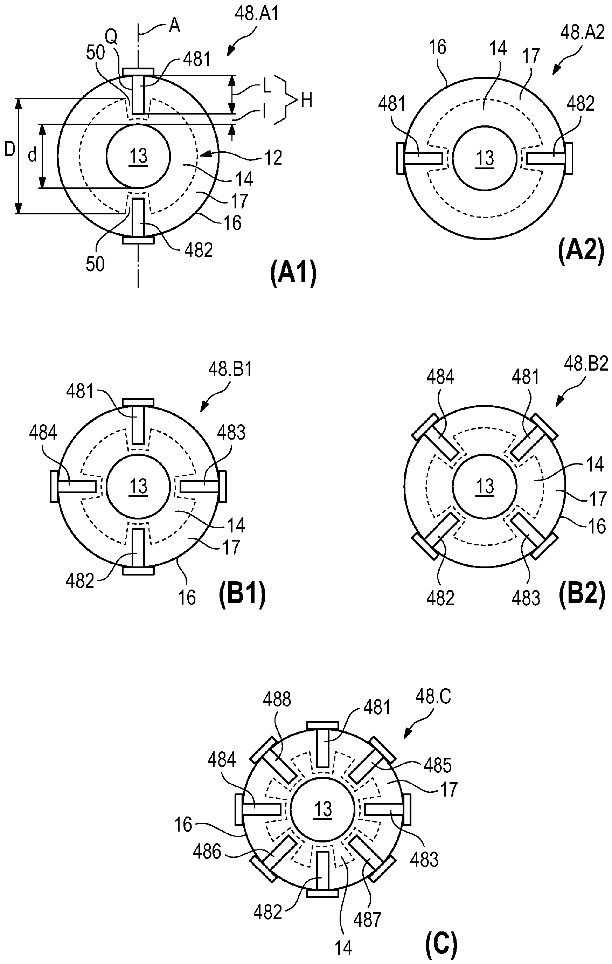

Das aus der Ausgangsöffnung 30 abgeführte SAP-Polymergel 44 mit geänderter Morphologie wird einem Trocknungsverfahren 46 zugeführt. Das Trocknungsverfahren 46 dient dazu dem SAP-Polymergel 44 mit geänderter Morphologie die Flüssigkeit zu entziehen, um dieses zu trocknen. Es sind verschiedene Trocknungsverfahren 46 denkbar und dem Fachmann bekannt. Im Rahmen des in Fig.4 beschriebenen Verfahrens wird ein mögliches Trocknungsverfahren 46 näher erläutert. Dem Trocknungsverfahren 46 können noch weitere Verfahrensschritte folgen (nicht gezeigt).

Nachfolgend werden weitere Details der Schnecke 14 und Welle 13 der Schneckenwelle 12 dieses Ausführungsbeispiels des Einwellenextruders 10 erläutert.

Die Schnecke 14 der Schneckenwelle 12 hat eine entlang der Förderzone 18 des Leitungskanals 16 zunehmende Ganghöhe G. In diesem Ausführungsbeispiel nimmt die Ganghöhe kontinuierlich zu. Alternativ, jedoch weniger bevorzugt, könnte die Ganghöhe zumindest entlang eines Abschnitts der Förderzone 18 auch abnehmen (nicht gezeigt). Die Ganghöhe kann auch erst zunehmen und danach wieder abnehmen oder zuerst abnehmen und danach wieder zunehmen (nicht gezeigt). Die Ganghöhe kann sich auch diskontinuierlich bzw. stufenweise verändern, beispielsweise bevorzugt diskontinuierlich bzw. stufenweise zunehmen (nicht gezeigt). Zwischen den jeweiligen gezeigten Abschnitten der Schneckenwindungen 22 der Schnecke 14 ist erkennbar, dass sich die unterschiedlich großen Ganghöhenwerte G1, G2, G3 und G4 ergeben.

Der erste Ganghöhenwert G1 der Ganghöhe beträgt in diesem Ausführungsbeispiel für die Laborversion des Einwellenextruders 46 mm und der vierte Ganghöhenwert G4 beträgt 70 mm, so dass die Ganghöhe G entlang der Förderzone von einem Ganghöhenwert von 46 mm nahe der Eingangszone auf einen Ganghöhenwert von 70 mm nahe der Ausgangszone zunimmt.

Für die Produktionsversion des Einwellenextruders 10 beträgt der erste Ganghöhenwert G1=500 mm und der vierte Ganghöhenwert G4 beträgt 650 mm. Der zweite und dritte Ganghöhenwert G2 und G3 liegen zwischen diesen Werten. Für die Laborversion beträgt G2=54 mm und G3=62 mm.

Für die Produktionsversion dieses Ausführungsbeispiels beträgt G2=550 mm und G3=600 mm. Die Ganghöhen G können in einem alternativen nicht gezeigten Ausführungsbeispiel beispielsweise auch zwischen Ganghöhenwerten von 30 mm und 800 mm liegen.

Die unterschiedlichen Ganghöhenwerte G1, G2, G3, G4 der Ganghöhe gehen mit unterschiedlich steilen Helixwinkeln φ1, φ2, φ3 und φ4 einher (nicht gezeigt).

Der Wert der Laufbreite e der Schnecke 14 beträgt in diesem Ausführungsbeispiel für die Laborversion des Einwellenextruders 10 konstante 6 mm und für die Produktionsversion des Einwellenextruders 10 konstante 40 mm. Die Laufbreite der Schnecke 14 kann alternativ auch beispielsweise Werte zwischen 4 mm und 80 mm haben und sich entlang des Leitungskanals 16 verändern, beispielsweise zunehmen oder abnehmen, insbesondere kontinuierlich zunehmen oder abnehmen.

In diesem Ausführungsbeispiel hat die Welle 13 der Schneckenwelle 12 jedenfalls entlang der Förderzone 18, vorzugsweise zusätzlich auch über die Eingangszone 26 und/oder Ausgangszone 28, einen konstanten Wellendurchmesser d. Der konstante Wellendurchmesser d hat hier einen Wert von 35 mm für die Laborversion des Einwellenextruders 10 bzw. der konstante Wellendurchmesser d hat hier einen Wert von 340 mm für die Produktionsversion des Einwellenextruders 10. Insbesondere kann der Wellendurchmesser d auch Werte zwischen 25 mm und 500 mm haben, beispielsweise zwischen 340 mm und 380 mm haben.

Der Wert des Schneckenaußendurchmesser D beträgt in diesem Ausführungsbeispiel 78 mm für die Laborversion des Einwellenextruders 10 bzw. 650 mm für die Produktionsversion des Einwellenextruders 10. Insbesondere kann der Schneckenaußendurchmesser D auch Werte zwischen 60 mm und 1000 mm, beispielsweise zwischen 600 mm und 700 mm haben. In diesem Ausführungsbeispiel ist das Verhältnis des Wellendurchmessers d zu Schneckenaußendurchmesser D konstant und beträgt ungefähr 0,449 für die Laborversion des Einwellenextruders 10 und 0,52 für die Produktionsversion des Einwellenextruders 10. Insbesondere kann das Verhältnis des Schneckenwellendurchmessers d zu Schneckenaußendurchmesser D auch zwischen 0,4 und 0,6 liegen, beispielsweise zwischen 0,449 und 0,577 oder insbesondere auch zwischen 0,52 und 0,58.

Der Wert der Förderraumhöhe H zwischen der Welle 13 der Schneckenwelle 12 und einer Wand des Leitungskanals 16 beträgt in diesem Ausführungsbeispiel 43 mm für die Laborversion des Einwellenextruders 10 und 310 mm für die Produktionsversion des Einwellenextruders 10. Alternativ kann die Förderraumhöhe H auch Werte zwischen 25 mm und 500 mm, beispielsweise zwischen 270 mm und 310 mm haben.

Das Ausführungsbeispiel der Laborversion des Einwellenextruders 10 basiert auf einem ECT EXR T80. In der nachfolgenden Tabelle 2 findet sich eine Parameterübersicht des Ausführungsbeispiels der Laborversion des Einwellenextruders 10 und der Produktionsversion des Einwellenextruders 10, sowie Angaben zu bevorzugten Parameterbereichen für Einwellenextruder 10.

**Tabelle 2:**

| Parameter | ECT EXR T80 (Laborversion) | Einwellenextruder (Produktionsversion) | Parameterbereich |
|---|---|---|---|
| Schneckenarbeitslänge (SAL) | 300 mm | 3000 mm | 200 mm bis 4000 mm |
| Leitungskanalinnendurchmesser (LID) | 80 mm | 650 mm | 50 mm bis 750 mm |
| LID/SAL | 3,75 | 4,6 | 2,22 bis 8 |
| Maximaler Druck | 50 bar | 50 bar | 30 bis 70 bar |
| Umdrehungsgeschwindigkeit im Betrieb | 75 Umdrehungen/Minute | 30 Umdrehungen/Minute | 15 U/Min. bis 150 U/Min |
| Düsendicke | 33 mm | 33 mm | 20 mm bis 45 mm |
| Düsenlochdurchmesser | 8 mm | 8 mm | 4 mm bis 12 mm |
| Anzahl der Düsenlöcher | 66 | 3600 | 1 bis 4000 |
| Relativ offene Fläche der Düse | 66% | 10% | 5% bis 66% |
| Max. Durchsatz | -340 kg/h | 30 t/h | 200 kg/h bis 30 t/h |
| Max. spezifische mechanische Energieaufnahme | -60 kWh/t | -60 kWh/t | 20 kWh/t bis 100 kWh/t |
| Temperatur des Öls der Heizvorrichtung | 150°C | ohne Ölerwärmung | 80°C bis 200°C |
| Temperatur im Leitungskanal | ~85-95°C | 95-140 °C | |
| SAP-Polymergeltemperatur an der Ausgangsöffnung | ~78-90°C | 90-110°C | |

Der Einwellenextruder 10 ist nicht auf die in Tabelle 2 angegebenen Parameter beschränkt, diese stellen lediglich einen bevorzugten Parameterbereich dar.

Fig.3 zeigt ein weiteres bevorzugtes Ausführungsbeispiel eines Einwellenextruders 10. Das weitere bevorzugte Ausführungsbeispiel des Einwellenextruders 10 betrifft vorzugsweise eine Produktionsversion. Der Einwellenextruder 10 kann grundsätzlich auch als Laborversion ausgebildet sein (nicht gezeigt). Der Einwellenextruder 10 des weiteren bevorzugten Ausführungsbeispiels ist ähnlich zu dem in Fig.2 gezeigten Ausführungsbeispiel des Einwellenextruders 10 - es sind somit für ähnliche oder gleiche Teile oder Teile ähnlicher oder gleicher Funktion der Einfachheit halber gleiche Bezugszeichen verwendet. Die Beförderungsrichtung 20 entlang der Förderzone 18 verläuft in dieser Darstellung wiederum von links nach rechts.

Der Einwellenextruder 10 hat eine Eingangsöffnung 36, einen Leitungskanal 16, eine Schneckenwelle 12 und eine Ausgangsöffnung 30. Der Leitungskanal 16 hat einen Innenraum 17, dem eine Eingangszone 26, eine Förderzone 18 und eine Ausgangszone 28 zugeordnet ist und der sich zwischen der Eingangsöffnung 36 und der Ausgangsöffnung 30 erstreckt. Der Einwellenextruder 10 kann verwendet werden, um ein superabsorbierendes Polymergel (SAP-Polymergel) 24 in Beförderungsrichtung 20 zu fördern und eine Morphologie des SAP-Polymergels 24 zu ändern.

Auch in diesem Ausführungsbeispiel ist der Leitungskanal 16 des Einwellenextruders 10 vollständig von einer Heizvorrichtung 42 umschlossen. Die Heizvorrichtung 42 kann mit einem Öl mit einer vorbestimmten Temperatur befüllt werden, um eine gewünschte Temperatur im Innenraum 17 des Leitungskanals 16 einzustellen.

Wie der Einwellenextruder 10 des Ausführungsbeispiels der Fig.2 wird auch der Einwellenextruder 10 des bevorzugten Ausführungsbeispiels der Fig.3 mit SAP-Polymergel 24 befüllt, das aus einem Polymerisationsverfahren 38 stammt. Dieses wird über die Eingangsöffnung 36 in den Innenraum 17 des Einwellenextruders 10 zugeführt und von den Schneckenwindungen 22 der Schnecke 14 der Schneckenwelle 12 entlang des Leitungskanals 16 in Beförderungsrichtung 20 gefördert.

Während der Beförderung entlang des Leitungskanals 16 wird die Morphologie des SAP-Polymergels geändert, insbesondere die Porosität der Oberfläche der Partikel des SAP-Polymergels erhöht. An der Ausgangsöffnung 30 wird das SAP-Polymergel durch die schematisch dargestellte Lochscheibe 32 und die Düse 34 gedrückt und die Morphologie weiter verändert, so dass ein SAP-Polymergel 44 mit geänderter Morphologie erzeugt wird. Das SAP-Polymergel 44 mit geänderter Morphologie wird dann einem Trocknungsverfahren 46 zugeführt. Es können noch weitere Verfahren nach dem Trocknungsverfahren 46 folgen (nicht gezeigt).

Das weitere bevorzugte Ausführungsbeispiel des Einwellenextruders 10 der Fig.3 unterscheidet sich im Wesentlichen dadurch von dem Ausführungsbeispiel des Einwellenextruders 10 der Fig.2, dass der Einwellenextruder 10 der Fig.3 eine Mischelementanordnung 49 mit jeweils zwei hier gezeigten Mischelementen in Form von Stiften 48.1 und 48.2 einer Stiftanordnung 48 aufweist, und sich der Wellendurchmesser d der Welle 13 entlang des Leitungskanals 16 ändert - in diesem beispielhaften Fall zunimmt. Hier hat die Welle 13 der Schneckenwelle 12 jedenfalls entlang der Förderzone 18, vorzugsweise zusätzlich auch über die Eingangszone 26 und/oder Ausgangszone 28, einen zunehmenden Wellendurchmesser d.

Ein Ausführungsbeispiel eines Einwellenextruders 10 der Fig.2 --mit Mischelementanordnung 49 und konstantem Wellendurchmesser d-- ist hier nicht explizit gezeigt aber ebenfalls gemäß dem allgemeinen Konzept der Erfindung.

Beim zweiten Ausführungsbeispiel des Einwellenextruders 10 der Fig.3 weist zudem die Mischelementanordnung 49 hier vier Stiftanordnungen 48 mit hier gezeigten Stiften 48.1, 48.2 im Leitungskanal 16 --und ggfs. Aussparungen 50 in der Schnecke 14 der Schneckenwelle 12-- auf. Varianten der Stiftanordnungen 48 sind in Fig.4 beispielhaft erläutert.

Die Stifte 48.1, 48.2 sind in diesem Ausführungsbeispiel des Einwellenextruders 10 in der Wand des Leitungskanals 16 befestigt und ragen in den Innenraum 17 des Leitungskanals 16. Die Stifte 48.1, 48.2 einer Stiftanordnung sind um den gesamten Umfang des Leitungskanals 16 angeordnet, so dass sich um den Umfang herum ein Ring aus Stiften bildet, von denen hier zwei Stifte 48.1, 48.2 gezeigt sind. Die Stifte 48.1, 48.2 einer Stiftanordnung sind dabei derart angeordnet, dass sie einen kleinen Abstand entlang des Umfangs voneinander haben, so dass die Stiftringe aus Stiften 48.1, 48.2 einer oder mehrerer Stiftanordnung 48 insgesamt eine gitterartige Struktur bzw. ein Gitter einer Mischelementanordnung 49 bilden.

Zwischen jeweils zwei der entlang der Beförderungsrichtung 20 mit einem Mischelementabstand K getrennt angeordneten Stiftringe aus Stiften einer Stiftanordnung 48 bildet sich praktisch eine Leitungskanalkammer aus.

Die Länge der jeweiligen Leitungskanalkammer ist durch den Wert der Stiftabstände K1, K2, K3, K4, K5 definiert und in diesem Ausführungsbeispiel unterschiedlich, da die Stiftanordnungen 48 mit unterschiedlichen Stiftabständen K angeordnet sind. Die erste Leitungskanalkammer entlang der Beförderungsrichtung 20 hat einen Wert des Mischelementabstands K1 und ist von einer Wand des Leitungskanals 16 und dem ersten Stiftring einer Stiftanordnung 48 mit Stiften 48.1 und 48.2 gebildet. Die erste Leitungskanalkammer erstreckt sich von der Eingangszone 26 in die Förderzone 18. Die in Beförderungsrichtung 20 folgenden Leitungskanalkammern sind zwischen den jeweiligen Stiftringen einer weiteren nicht näher bezeichneten aber erkennbaren Stiftanordnung 48 aus weiteren nicht näher bezeichneten aber erkennbaren Stiften 48.1, 48.2 in der Förderzone 18 gebildet und haben für die Stiftabstände die Werte K2, K3 und K4. Die Leitungskanalkammer an der Ausgangszone 28 des Leitungskanals 16 wird zwischen der letzten Stiftanordnung 48 in Beförderungsrichtung 20 und der Lochscheibe 32 über einen Stiftabstand K5 gebildet.

Die in Fig. 4 erkennbaren Aussparungen 50 in der Schnecke 14 sind derart angeordnet, dass im Betrieb, wenn die Welle 13 über den Antriebsmotor 40 rotiert wird, die Stifte 48.1, 48.2 die Aussparungen 50 während der Drehung der Welle 13 passieren, ohne, dass die Stifte 48 die Schnecke 14 berühren. Die Aussparungen 50 stellen also Unterbrechungen in der Schnecke 14 dar.

Die Stifte 48.1, 48.2 einer Stiftanordnung 48 und die Aussparungen 50 dienen der Durchmischung und der Scherung des SAP-Polymergels 24. Eine verbesserte Durchmischung und Erhöhung der Scherung zeigt sich insbesondere für eine Produktionsversion des Einwellenextruders 10, d.h. eine Version mit größeren Dimensionen. Für eine Laborversion mit kleineren Dimensionen wirkt sich die Mischelementanordnung 49 schwächer aus.

In diesem Ausführungsbeispiel des Einwellenextruders 10 ist die Welle 13 der Schneckenwelle 12 in fünf Wellensegmente W1, W2, W3, W4 und W5 eingeteilt. Das Wellensegment W1 liegt in der Eingangszone 26, die Wellensegmente W2, W3 und W4 liegen in der Förderzone 18 und das Wellensegment W5 liegt in der Ausgangszone 28. Die Wellensegmente W1 bis W5 haben einen Wellendurchmesser d mit einem ersten Wert d1=340 mm. Das Wellensegment W3 hat einen kontinuierlich von dem ersten Wert d1=340 mm auf einen zweiten Wert d2=380 mm --d.h. um Δd-- sich ändernden Wellendurchmesser. In den Wellensegmenten W4 und W5 hat der Wellendurchmesser zunächst den Wert von d2=380 mm und bleibt dann bei d2 bzw. (wie hier) steigt dann weiter an auf d3>380 mm an. Im Wellensegment W5, d.h. in der Ausgangszone 28 verringert sich der Wellendurchmesser d kontinuierlich von dem Wert d2=340 mm bzw. d3>380 auf einen Wert von d4=120mm.

Die Ausgangszone 28 hat somit ein größeres Transportvolumen, was es ermöglicht eine größere Menge SAP-Polymergel unter geringerem Druck durch die Ausgangsöffnung 30 zu drücken.

Die Schneckenwelle 12 hat in diesem Ausführungsbeispiel einen niedrigsten Wert eines Wellendurchmessers d1 entlang des Leitungskanals 16 der ca. 32% des höchsten Wertes des Wellendurchmessers d2 bzw. d3 entlang des Leitungskanals 16 beträgt. Der niedrigste Wert des Wellendurchmessers d entlang des Leitungskanals 16 kann auch höchstens zwischen 20% und 80% eines höchsten Wertes eines Wellendurchmessers entlang des Leitungskanals 16 betragen. Der Wert des Schneckenaußendurchmessers D ist konstant entlang des Leitungskanals 16 und beträgt 650 mm. Somit beträgt das Verhältnis d/D des Wellendurchmessers d zum Schneckenaußendurchmesser D d/D=0,52 entlang der Förderzone 18 des Leitungskanals 16.

Die Ganghöhe G der Schnecke 14 nimmt in der hier dargestellten Ausführungsform von einem ersten Ganghöhenwert G1=500 mm über G2=550 mm auf G3=600 mm zu, d.h. der niedrigste Ganghöhenwert am Beginn der Förderzone 18 entlang des Leitungskanals kann 83% des höchsten Ganghöhenwertes am Ende der Förderzone 18 betragen. Der niedrigste Ganghöhenwert G1 entlang des Leitungskanals kann auch höchstens zwischen 20% und 85% des höchsten Ganghöhenwertes G3 entlang des Leitungskanals betragen. In den Wellensegmenten W2 zu W3 kann die Schnecke 14 den zweiten Ganghöhenwert G2=550 mm haben. In den Wellensegmenten W3 zu W4 kann sich also die Ganghöhe G auf G3 erhöhen. Dies führt zu einer Druckerniedrigung und Transportvolumenvergrößerung entlang des Leitungskanals 16 in der Beförderungsrichtung 20 bereits im Bereich der Wellensegmente W2, W3 und W4.

Die Ganghöhe kann in einer hier nicht gezeigten alternativen Ausführungsform auch stufenweise abnehmen von einem ersten Ganghöhenwert G1=500 mm - im Wellensegment W2 kann die Schnecke 14 den zweiten Ganghöhenwert G2=460 mm haben. Es kann also in einem hier nicht gezeigten Ausführungsbeispiel die Ganghöhe auch abnehmen, von einem ersten Ganghöhenwert G1=500 mm über G2=460 mm auf G3= 350 mm abnehmen und dann ggfs. weiter abnehmen auf einen Wert G4<350 mm, d.h. der niedrigste Ganghöhenwert entlang des Leitungskanals kann auch 70% des höchsten Ganghöhenwertes betragen am Ende der Förderzone 18 oder sogar darunter liegen. Der niedrigste Ganghöhenwert entlang des Leitungskanals kann am Ende der Förderzone 18 auch höchstens zwischen 20% und 80% des höchsten Ganghöhenwertes entlang des Leitungskanals am Beginn der Förderzone 18 betragen.

In einer hier nicht gezeigten alternativen Ausführungsform verringert sich also vor allem im Wellensegment W3 einerseits die Ganghöhe G und andererseits erhöht sich der Wellendurchmesser d. Dies führt zu einer Druckerhöhung und Transportvolumenverringerung entlang des Leitungskanals 16 in der Beförderungsrichtung 20. Dies kann in einer ersten Variante so beibehalten werden über die Wellensegmente W4 und W5 (nicht gezeigt) oder weiter in der Tendenz verstärkt werden in den Wellensegmenten W4 und W5 durch weitere Erhöhung des Wellendurchmessers d (auf d2 und dann d3) und weitere Verringerung der Ganghöhe G (auf beispielsweise einen Wert G4 und dann G5 - nicht gezeigt).

Die Werte des Mischelementabstands K2, K3, K4 und K5 sind in dem in Fig.3 gezeigten Ausführungsbeispiel auf die Ganghöhenwerte G2, G3 der Ganghöhe G der Schnecke 14 gegenläufig angepasst. Während sich die Werte der Stiftabstände von K2 auf K5 verringert, erhöhen sich die Ganghöhenwerte von G1 auf G3. Die Änderung des Mischelementabstands K verläuft also in eine gegenläufige Richtung wie die Änderung der Ganghöhe G. Dies führt zu einer eher geringeren Einbringung einer Druckerhöhung und Transportvolumenverringerung in Folge der zunehmenden Ganghöhe G aber einer verbesserten Durchmischung und Erhöhung der Scherung in Beförderungsrichtung 20 zur Ausgangszone 28 des Einwellenextruders 10 hin in Folge des abnehmenden Stiftabstandes K.

Es kann in einer hier nicht gezeigten Abwandlung die Änderung des Mischelementabstands K auch gleichläufig zur Änderung der Ganghöhe G ausgebildet sein. Mit den Bezugszeichen der Fig.3 wären die Werte des Mischelementabstands K2, K3, K4 und K5 in einem solchen abgewandelten Ausführungsbeispiel auf die Ganghöhenwerte G1, G2 und G3 der Ganghöhe G der Schnecke 14 gleichläufig angepasst. Während sich die Werte der Stiftabstände in dem Fall der abgewandelten Ausführungsform von K1 auf K5 erhöhten (nicht gezeigt), erhöhten sich gleichläufig auch die Ganghöhenwerte von G1 auf G3 (gezeigt). Dies führte zu einer vergleichsweise moderaten Einbringung von Druck- und Scherkräften in Folge der eher vermiedenen Druckerhöhung und Transportvolumenverringerung und andererseits einer verbesserten aber nicht zunehmenden Durchmischung, d.h. einer eher nachlassenden Scherung in Beförderungsrichtung 20 zur Ausgangszone 28 des Einwellenextruders 10 hin.

Die nachfolgende Tabelle 3 fasst die Parameter des Ausführungsbeispiels einer Laborversion und einer Produktionsversion einer Schneckenwelle 12 analog der Fig.3 zusammen.

**Tabelle 3:**

| Eigenschaft der Schneckenwelle und Schnecke | Ausführungsbeispiel der Schneckenwelle der Fig.3 (Laborversion) | Ausführungsbeispiel der Schneckenwelle der Fig.3 (Produktionsversion) |
|---|---|---|
| Gangweite (W) | W | W |
| Laufbreite (e) | 6 mm | 40 mm |
| Ganghöhe (G=π(D-e)tanφ) | 46 mm zu 70 mm | 500mm zu 600mm |
| Schneckenaußendurchmesser (D) | 78 mm | 650 mm |
| Förderraumhöhe (H) | 43 mm | 310 mm |
| Wellendurchmesser (d) (soweit konstant) | 35 mm | 340mm |
| d/D | 0,45 | 0,52 |
| Abstand zwischen Gehäusewand und Schnecke (δ) | δ | 5δ |
| Helixwinkel (φ) | φ1 zu φ2 | φ1 zu φ2 |

Fig.4 zeigt in Ansichten (A1, A2), (B1, B2) und (C) bevorzugte Varianten von Stiftanordnungen 48 mit Stiften für eine in Fig.3 gezeigte Mischelementanordnung; die Stiftanordnungen sind entsprechend mit 48.A1, 48.A2, 48.B1, 48.B2 und 48.C bezeichnet. Die Stiftanordnungen 48.A1 und 48.A2 haben jeweils zwei auf einer diametralen Achse A gegenüberliegend angeordnete Stifte 481, 482. Die Stiftanordnungen 48.A1 und 48.A2 unterscheiden sich in der Ausrichtung der diametralen Achse mit den Stifte 481, 482 jeweils an einer 12-Uhr-Position und 6-Uhr-Position (Ansicht A1) bzw. mit den Stifte 481, 482 jeweils an einer 3-Uhr-Position und 9-Uhr-Position (Ansicht A2).

In Ansicht A1 ist zudem beispielhaft die Länge L eines Stiftes 481, 482 im Bereich von hier etwa L=300mm und der Abstand I eines Stiftendes von der Oberfläche der Welle 13 482 im Bereich von hier etwa l=10mm angegeben; letzterer Abstand I kann beispielsweise im Bereich von 10-25mm liegen. Die Länge L eines Stiftes 481, 482 kann im Bereich von etwa L=270mm bis 330mm liegen.

Damit liegt die in den Innenraum 17 des Leitungskanals 16 ragende Länge L des Stiftes vorzugsweise bei etwa 90% bis 99% der freien Förderraumhöhe H des Leitungskanals 16. Die Förderraumhöhe H gibt den Abstand zwischen Wand des Leitungskanals 16 und Oberfläche der Welle 13 an und liegt hier bei 310mm. Der Durchmesser Q eines vorzugsweise runden (ggfs. jedoch auch möglichen ovalen oder eckigen) Querschnittes eines Stiftes liegt bei etwa Q= 1 - 8cm, vorzugsweise 2 - 6cm, besonders vorzugsweise 4cm. Diese Stiftanordnungen 48.A1 und 48.A2 können auch für eine Mischelementanordnung 49 entlang der Beförderungsrichtung des Einwellenextruders 10 kombiniert werden, sich z.B. abwechseln.

Die Stiftanordnungen 48.B1 und 48.B2 haben jeweils vier auf einer ersten und zweiten diametralen Achse A1, A2 sich paarweise gegenüberliegend angeordnete Stifte 481, 482 und 483, 484. Die Stiftanordnungen 48.B1 und 48.B2 unterscheiden sich in der Ausrichtung der diametralen Achsen A1, A2 mit den Stifte 481, 482, 483, 484 jeweils an einer 12-Uhr-Position und 6-Uhr-Position sowie 3-Uhr-Position und 9-Uhr-Position (Ansicht B1) bzw. mit den Stifte 481, 482, 483, 484 jeweils an einer 2-Uhr-Position und 8-Uhr-Position sowie 5-Uhr-Position und 11-Uhr-Position (Ansicht B2). Diese Stiftanordnungen 48.B1 und 48.B2 können auch für eine Mischelementanordnung 49 entlang der Beförderungsrichtung des Einwellenextruders 10 kombiniert werden, sich z.B. abwechseln. Die Maße der Stifte der Stiftanordnungen 48.B1 und 48.B2 können wie die bei den Stiften der Stiftanordnungen 48.A1 und 48.A2 gewählt werden.

Die Stiftanordnung 48.C hat acht sich paarweise gegenüberliegend angeordnete Stifte 481, 482 und 483, 484 sowie 485, 486 und 487, 488 - es handelt sich praktisch um eine Überlagerung der Stiftanordnungen 48.B1 und 48.B2. Diese Stiftanordnung 48.C kann auch für eine Mischelementanordnung 49 entlang der Beförderungsrichtung des Einwellenextruders 10 kombiniert werden mit den Stiftanordnungen 48.B1 und 48.B2 und/oder Stiftanordnungen 48.A1 und 48.A2.

Grundsätzlich können auch andere Winkelanordnungen für die Ausrichtung der Stifte in einer Stiftanordnung 48 für eine Mischelementanordnung 49 gewählt werden.

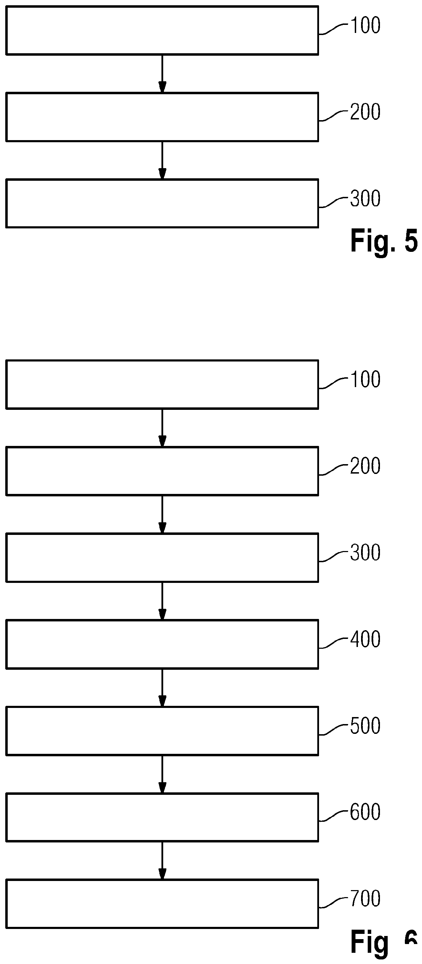

Fig.5 zeigt ein erstes bevorzugtes Ausführungsbeispiel eines Verfahrens zum Ändern der Morphologie eines superabsorbierenden Polymergels (SAP-Polymergel) 24 mit einem Einwellenextruder 10 der bevorzugten Ausführungsbeispiele der Fig.2 (mit dort nicht gezeigten aber vorhandenen Mischelementen einer Mischelementanordnung 49) oder Fig.3 (mit den dort gezeigten Mischelementen einer Mischelementanordnung 49), d.h. jeweils mit Mischelementanordnungen 49. Das Verfahren weist die Schritte auf:

| | |
|---|---|
| 100 | Zuführen des SAP-Polymergels 24 von einem Polymerisationsverfahren 38 in den Einwellenextruder 10, |
| 200 | Betreiben des Einwellenextruders 10, um die Morphologie des SAP-Polymergels 24 zu ändern und |
| 300 | Abführen des SAP-Polymergels 44 mit geänderter Morphologie aus dem Einwellenextruder 10 zu einem Trocknungsverfahren 46. |

Das Polymerisationsverfahren des Schrittes 100 erzeugt ein sogenanntes Basispolymer als Basis für das SAP-Polymergel 24 nach der Formulierung der Tabelle 1. Es können alternativ auch andere Formulierungen verwendet werden.

Der Einwellenextruder 10 wird gemäß Schritt 200 betrieben, indem der Antriebsmotor 40 die Welle 13 der Schneckenwelle 12 rotiert und SAP-Polymergel 24 von der Eingangsöffnung 36 zur Ausgangsöffnung 30 befördert wird. Während das SAP-Polymergel 24 entlang der Förderzone 18 gefördert wird, kann die Morphologie des SAP-Polymergels 24 geändert werden. Die Förderzone 18 dient auch als Zuführungszone zur Ausgangszone 28, die als Dosierungszone für die Ausgangsöffnung 30 dient. Je nach Zufuhr von SAP-Polymergel 24 zur Ausgangszone 28, die u.a. von den Parametern der Schneckenwelle 12 und ihrer Umdrehungsgeschwindigkeit abhängt, wird eine bestimmte Menge an SAP-Polymergel 24 in der Ausgangszone 28 bereitgestellt. Diese bestimmte Menge kann dann mit einem bestimmten Druck durch die Ausgangsöffnung 30 herausgeführt werden. Die Ausgangsöffnung 30 hat formgebende Komponenten, die die Morphologie des SAP-Polymergels 24 ändern. In dem Ausführungsbeispiel des Verfahrens nach Fig.5 weist die Ausgangsöffnung 30 eine Lochscheibe 32 und eine nachfolgende Düse 34 auf. Alternativ oder zusätzlich können auch andere Komponenten, wie eine Matrix oder andere die Morphologie des SAP-Polymergels 24 ändernde Komponenten an der Ausgangsöffnung 30 angeordnet sein.

Das Trocknungsverfahren 46 gemäß Schritt 300 hat in diesem Ausführungsbeispiel des Verfahrens der Fig.5 die folgenden Schritte:
- Verteilen des SAP-Polymergels 44 mit geänderter Morphologie auf Trocknungsblechen und
- Erhitzen des SAP-Polymergels 44 mit geänderter Morphologie auf den Trocknungsblechen in einem Konvektionsofen bzw. Umluftofen.

Die Trocknungsbleche können mit verschiedenen Mengen an SAP-Polymergel 44 mit geänderter Morphologie befüllt werden, so dass sich ein Befüllungsgrad in Abhängigkeit der Fläche des Trocknungsblechs und der Menge des auf das Trocknungsblech befüllten SAP-Polymergels 44 mit geänderter Morphologie ergibt. Die Trocknungstemperatur und Trocknungsdauer hängen von dem Befüllungsgrad ab. In diesem Ausführungsbeispiel wird bei einer Befüllung mit 0,91 g/cm² eine Trocknungstemperatur von 175°C für 70 Minuten zum Trocknen verwendet. Die Trocknungsbleche haben in diesem Ausführungsbeispiel einen Siebboden mit 250 µm Maschenöffnungen, so dass die Flüssigkeit auch durch den Siebboden entweichen kann. Der Siebboden ist in diesem Ausführungsbeispiel aus Drähten mit einem Durchmesser von jeweils 130 µm gebildet.

Fig.6 zeigt ein zweites bevorzugtes Ausführungsbeispiel eines Verfahrens zum Ändern einer Morphologie eines superabsorbierendem Polymergels (SAP-Polymergel) 24 mit einem Einwellenextruder 10 der bevorzugten Ausführungsbeispiele der Fig.2 oder Fig.3 jeweils mit Mischelementanordnungen 49. Das Verfahren weist die Schritte auf:

| | |
|---|---|
| 100 | Zuführen des SAP-Polymergels 24 von einem Polymerisationsverfahren 38 in den Einwellenextruder 10. |
| 200 | Betreiben des Einwellenextruders 10, um die Morphologie des SAP-Polymergels 24 zu ändern. |
| 300 | Abführen des SAP-Polymergels 44 mit geänderter Morphologie aus dem Einwellenextruder 10 zu einem Trocknungsverfahren 46. |
| 400 | Zuführen des getrockneten SAP zu einem Mahlverfahren, um das SAP zu mahlen. |
| 500 | Zuführen des gemahlenen SAP zu einem Siebverfahren bzw. Sortierverfahren, um das SAP zu sieben bzw. zu sortieren. |
| 600 | Zuführen von sortieren SAP-Partikeln zu einem Mischverfahren, um die SAP-Partikel zu mischen. |
| 700 | Zuführen der SAP-Partikel-Mischung zu einem Oberflächen-Nachvernetzungsverfahren bzw. surface cross linking (SXL)-Verfahren, um eine Oberflächen-Nachvernetzung auf den Oberflächen der SAP-Partikel durchzuführen. |

Die Schritte 100, 200 und 300 des Verfahrens sind identisch zu den in Fig.5 beschriebenen Schritten des Ausführungsbeispiels des Verfahrens zum Ändern der Morphologie eines superabsorbierenden Polymers, nämlich eines Polymergels (SAP-Polymergels) 24 mit einem Einwellenextruder 10. Das getrocknete SAP wird im Mahlverfahren in Schritt 400 von einer Mühle gemahlen - es können SAP-Partikel mit unterschiedlichen Größen entstehen. Das gemahlene SAP wird im Siebverfahren bzw. Sortierverfahren des Schrittes 500 gesiebt bzw. sortiert, so dass SAP-Partikel mit verschiedenen Größen voneinander getrennt werden. Im Mischverfahren des Schrittes 600 werden SAP-Partikel verschiedener Größen entsprechend einem vorbestimmten Mischverhältnis gemischt, um gewünschte Eigenschaften der SAP-Partikel-Mischung zu erhalten. In diesem Ausführungsbeispiel wurde eine SAP-Partikel-Mischung mit einer Partikelgrößenverteilung (particle size distribution - psd) entsprechend der Tabelle 4 erzeugt.

**Tabelle 4:**

| Siebbereich | > 710 µm | 710-600 µm | 600-500 µm | 500-300 µm | 300-200 µm | 200-150 µm | < 150 µm | Gesamt |
|---|---|---|---|---|---|---|---|---|
| Anteil (Gewichts-%) | 0 | 10,6 | 27,9 | 42,7 | 13,8 | 5,0 | 0 | 100,0 |

Im Oberflächen-Nachvernetzungsverfahren (SXL-Verfahren) des Schrittes 700 wird auf die Oberfläche der SAP-Partikel ein Oberflächenvernetzungsstoff aufgebracht. Der Oberflächenvernetzungsstoff in diesem Ausführungsbeispiel wurde gemäß der in der nachfolgenden Tabelle 5 gezeigten Formulierung hergestellt:

**Tabelle 5:**

| | |
|---|---|
| flüssiger Anteil | 4,54 Gewichts-% baP (Isopropanol/Wasser=30,9/69,1) |
| Heonon/1,3-PG | 0,07/0,07 Gewichts-% baP |
| 1,2-PG | 0,7 Gewichts-% baP |
| Isopropanol | 0,99 Gewichts-% baP |
| Span 20 | 40 ppm baP |
| Aluminiumlactat | 0,5 Gewichts-% baP (als 22 Gewichts-% flüssige Lösung Lohtragon AL 220) |
| Temperatur | 185°C |

Heonon entspricht 2-Hydroxyethyl-2-oxazolidinon. Alternativ zur Formulierung in Tabelle 5 können auch andere Formulierungen für die Herstellung des Oberflächenvernetzungsstoffs verwendet werden. Beispielsweise können alternative Oberflächenvernetzungsstoffe Heonon, Isopropanol, Aluminiumlactat, und/oder Aluminiumsulfat enthalten.

Die mit Oberflächenvernetzungsstoff benetzten SAP-Partikel werden bei einer Temperatur von 185° C erwärmt, so dass sich eine Oberflächenvernetzung auf den jeweiligen SAP-Partikeln ausbildet. Die Oberflächenvernetzung ermöglicht es den SAP-Partikeln im aufgequollenen Zustand, d.h. wenn die SAP-Partikel Flüssigkeit aufgenommen haben und als Polymergel vorliegen, ihre Form zu behalten. Insbesondere kann durch die Oberflächenvernetzung das Haltevermögen bei absorbierter Flüssigkeit unter Druck gesteigert werden.

### Bezugszeichenliste

- 10: Einwellenextruder
- 12: Schneckenwelle
- 13: Welle
- 14: Schnecke
- 16: Leitungskanal
- 17: Innenraum des Leitungskanals
- 18: Förderzone
- 20: Beförderungsrichtung
- 22: Schneckenwindung
- 24: SAP-Polymergel
- 26: Eingangszone
- 28: Ausgangszone
- 30: Ausgangsöffnung
- 32: Lochscheibe
- 34: Düse
- 36: Eingangsöffnung
- 38: Polymerisationsverfahren
- 40: Antriebsmotor
- 42: Heizvorrichtung
- 44: SAP-Polymergel mit geänderter Morphologie
- 46: Trocknungsverfahren
- 48: Stiftanordnung
- 48.1, 48.2: Stifte
- 481, 482, 483, 484: Stifte
- 485, 486, 487, 488: Stifte
- 49: Mischelementanordnung
- 50: Aussparung
- A: diametrale Achse der Stifte
- G: Ganghöhe ( G=π(D-e)tanφ)
- G1, G2, G3, G4: Ganghöhenwert
- d, Δd: Wellendurchmesser
- d1, d2, d3: Wert des Wellendurchmessers
- D: Schneckenaußendurchmesser
- H: Förderraumhöhe
- L: Länge eines Stiftes
- I: Abstand eines Stiftes von der Oberfläche der Welle 13
- Q: Durchmesser eines Querschnittes eines Stiftes
- K: Mischelementabstand
- K1, K2, K3, K4: Wert des Mischelementabstandes
- W: Gangweite
- W1, W2, W3, W4, W5, W6: Wellensegment
- e: Laufbreite
- δ: Abstand zwischen Wand des Leitungskanals und Schnecke
- φ: Helixwinkel

## Patentansprüche

1. Einwellenextruder (10) zum Ändern einer Morphologie von superabsorbierendem Polymer, nämlich für ein superabsorbierendes Polymergel (SAP-Polymergel) (24), aufweisend:
- eine Eingangsöffnung (36) zum Zuführen von SAP-Polymergel (24),
- einen mit der Eingangsöffnung (36) verbundenen Leitungskanal (16),
- eine im Leitungskanal (16) angeordnete Schneckenwelle (12) zum Fördern und Ändern der Morphologie des SAP-Polymergels (24), und
- eine mit dem Leitungskanal (16) verbundene Ausgangsöffnung (30) zum Abführen des SAP-Polymergels (44) mit geänderter Morphologie,
wobei die Schneckenwelle (12) eine Welle (13) und eine an der Welle (13) angeordnete Schnecke (14) aufweist und angeordnet und ausgebildet ist das SAP-Polymergel (24) mittels der Schnecke (14) von der Eingangsöffnung (36) zur Ausgangsöffnung (30) zu befördern,
wobei der Leitungskanal (16) eine an oder nahe der Eingangsöffnung (36) angeordnete Eingangszone (26), eine an oder nahe der Ausgangsöffnung (30) angeordnete Ausgangszone (28) und eine sich entlang des Leitungskanals (16) von der Eingangszone (26) zur Ausgangszone (28) erstreckende Förderzone (18) aufweist, wobei
- die Schneckenwelle (12) einen Ganghöhenwert einer Ganghöhe (G) der Schnecke (14) entlang der Förderzone (18) des Leitungskanals (16) hat, und
- der Leitungskanal (16) wenigstens eine Mischelementanordnung (49) mit wenigstens einem Mischelement aufweist, das in den Leitungskanal (16) des Einwellenextruders hineinragt und das zum Mischen des SAP-Polymergels (24) ausgebildet ist,
**dadurch gekennzeichnet, dass**
- der Leitungskanal (16) wenigstens zwei entlang des Leitungskanal (16) in der Beförderungsrichtung (20) mit einem Mischelementabstand (K) voneinander getrennt angeordnete Stifte (48) aufweist, wobei ein Wert des Mischelementabstands (K1, K2, K3, K4) auf einen Ganghöhenwert (G1, G2, G3) der Ganghöhe (G) der Schnecke (14) gegenläufig angepasst ist, nämlich
der Mischelementabstand (K) derart gegenläufig auf die Ganghöhe (G) angepasst ist, dass ein kleinerer Wert des Mischelementabstands (K1, K2, K3, K4, K5) vorgesehen ist, wenn ein größerer Ganghöhenwert (G1, G2, G3) vorgesehen ist und ein größerer Wert des Mischelementabstands (K1, K2, K3, K4, K5) vorgesehen ist, wenn ein kleinerer Ganghöhenwert (G1, G2, G3) vorgesehen ist.

2. Einwellenextruder (10) nach Anspruch 1, wobei die Ausgangsöffnung (30) ebenfalls zum Ändern der Morphologie des SAP-Polymergels (24) ausgebildet ist.

3. Einwellenextruder (10) nach Anspruch 1 oder 2, wobei die Schneckenwelle (12) einen ersten Ganghöhenwert (G1) einer Ganghöhe (G) der Schnecke (14) entlang der Förderzone (18) des Leitungskanals (16) und einen in Beförderungsrichtung (20) folgenden zweiten Ganghöhenwert (G2) der Ganghöhe (G) der Schnecke (14) entlang der Förderzone (18) des Leitungskanals (16) hat, wobei der zweite Ganghöhenwert (G2) größer oder gleich dem ersten Ganghöhenwert (G1) ist, alternativ kleiner als der erste Ganghöhenwert (G1) ist.

4. Einwellenextruder (10) nach einem der Ansprüche 1 bis 3, wobei die Schneckenwelle (12) entlang des Leitungskanals (16) wenigstens drei unterschiedliche Ganghöhenwerte (G1, G2, G3, G4) der Schnecke (14) aufweist.

5. Einwellenextruder (10) nach einem der Ansprüche 1 bis 4, wobei die Ganghöhe (G) der Schnecke (14) entlang der Förderzone (18) des Leitungskanals (16) zunimmt, wobei die Schnecke (14) einen kleinsten Ganghöhenwert (G1) entlang der Förderzone (18) an einem Übergang von der Eingangszone (26) zur Förderzone (18) und einen größten Ganghöhenwert (G2) entlang der Förderzone (18) an einem Übergang von der Förderzone (18) zur Ausgangszone (28) hat.

6. Einwellenextruder (10) nach einem der Ansprüche 1 bis 5, wobei die Mischelementanordnung (49) wenigstens einen sich in einen Innenraum (17) des Leitungskanals (16) erstreckenden Stift (48.1, 48.2) einer Stiftanordnung (48) und wenigstens eine Aussparung (50) entlang der Schnecke (14) aufweist, wobei der wenigstens eine Stift (48.1, 48.2) sich in die wenigstens eine Aussparung (50) der Schnecke (14) erstreckt, wenn die Schnecke (14) im Betrieb rotiert wird, so dass ein Rotieren der Welle (13) der Schneckenwelle (12) möglich ist, ohne dass der Stift (48.1, 48.2) die Schnecke (14) berührt.

7. Einwellenextruder (10) nach einem der Ansprüche 1 bis 6, mit einem Verhältnis (d/D) zwischen dem Wellendurchmesser (d) zu einem Schneckenaußendurchmesser (D) zwischen 0,4 und 0,6.

8. Einwellenextruder (10) nach einem der Ansprüche 1 bis 7, wobei die Schneckenwelle (12) einen niedrigsten Ganghöhenwert (G1) entlang des Leitungskanals (16) hat, der höchstens zwischen 20% und 85% eines höchsten Ganghöhenwertes (G4) entlang des Leitungskanals (16) beträgt.

9. Einwellenextruder (10) nach einem der Ansprüche 1 bis 8, wobei der Einwellenextruder (10) ausgebildet ist ein SAP-Polymergel (24) mit einer erwünschten die Wasserabsorption des SAP erhöhenden Morphologie und/oder mit einem erhöhten Durchsatz, insbesondere von wenigstens 23 Tonnen pro Stunde, zu erzeugen.

10. Verfahren zum Ändern der Morphologie eines superabsorbierenden Polymergels (SAP-Polymergels) (24) mit einem Einwellenextruder (10) nach einem der Ansprüche 1 bis 9, wobei das Verfahren die Schritte aufweist:
- Zuführen des SAP-Polymergels (24) von einem Polymerisationsverfahren (38) in den Einwellenextruder (10),
- Betreiben des Einwellenextruders (10), um die Morphologie des SAP-Polymergels (24) zu ändern, und
- Abführen des SAP-Polymergels (44) mit geänderter Morphologie aus dem Einwellenextruder (10) zu einem Trocknungsverfahren (46).

11. Verfahren nach Anspruch 10, wobei der Einwellenextruder (10) einen Antriebsmotor (40) und/oder eine Heizvorrichtung (42) aufweist, wobei
- der Antriebsmotor (40) ausgebildet ist, die Welle (13) der Schneckenwelle (12) mit bis zu 150 Umdrehungen pro Minute zu rotieren, und/oder
- die Heizvorrichtung (42) ausgebildet ist, den Leitungskanal (16) zu erwärmen, so dass dieser eine Temperatur zwischen 85° C und 140° C jedenfalls in der Eingangszone (26) hat, und sich beim Betrieb des Einwellenextruders (10) die Temperatur des SAP-Polymergel (24) im Leitungskanal (16) entlang der Beförderungsrichtung (20) bis zu einer Ausgangszone (28) erhöht.

12. Verfahren nach Anspruch 11, wobei
- sich die Temperatur des SAP-Polymergel (24) im Leitungskanal (16) entlang der Beförderungsrichtung (20) bis zu einer Ausgangszone (28) um eine Temperatur im Bereich von 20° C bis 40° C erhöht, und/oder
- weiter direkt nach der Ausgangsöffnung (30) das SAP-Polymergel (24) eine wieder verringerte Temperatur des wieder abgekühlten SAP-Polymergel (24) zwischen 90° C und 110° C liegt.

13. Verwendung eines Einwellenextruders (10) nach einem der Ansprüche 1 bis 9, **dadurch gekennzeichnet, dass** der Einwellenextruder (10) ausgebildet ist zum Ändern einer Morphologie von superabsorbierendem Polymergel (SAP-Polymergel) (24).

## Claims

1. A single-screw extruder (10) for changing a morphology of superabsorbent polymer, specifically for a superabsorbent polymer gel (SAP polymer gel) (24), having:
- an input aperture (36) for the introduction of SAP polymer gel (24),
- connected to the input aperture (36), a channel (16),
- arranged in the channel (16), a screw (12) for conveying, and changing the morphology of, the SAP polymer gel (24) and
- connected to the channel (16), an output aperture (30) for the discharge of the SAP polymer gel (44) with changed morphology,
where the screw (12) has a shank (13) and has screw flights (14) arranged on the shank (13) and is arranged and configured to convey the SAP polymer gel (24) from the input aperture (36) to the output aperture (30) by means of the screw flights (14), where the channel (16) has an input zone (26) arranged at, or in the vicinity of, the input aperture (36), an output zone (28) arranged at, or in the vicinity of, the output aperture (30), and a conveying zone (18) extending along the channel (16) from the input zone (26) to the output zone (28), where
- the screw (12) has a pitch value of a pitch (G) of the screw flights (14) along the conveying zone (18) of the channel (16), and
- the channel (16) has at least one mixing-element arrangement (49) with at least one mixing element which protrudes into the channel (16) of the single-screw extruder and which is configured for the mixing of the SAP polymer gel (24),
wherein
- the channel (16) has at least two pins (48) arranged along the channel (16) in the conveying direction (20) and separated from one another by a mixing-element separation (K), and there is negative correlation between a value of the mixing-element separation (K1, K2, K3, K4) and a pitch value (G1, G2, G3) of the pitch (G) of the screw flights (14), namely
the negative correlation between the mixing-element separation (K) and the pitch (G) is such that a smaller value of the mixing-element separation (K1, K2, K3, K4, K5) is provided when a larger pitch value (G1, G2, G3) is provided, and a larger value of the mixing-element separation (K1, K2, K3, K4, K5) is provided when a smaller pitch value (G1, G2, G3) is provided.

2. The single-screw extruder (10) according to claim 1, where the output aperture (30) is likewise designed to change the morphology of the SAP polymer gel (24) .

3. The single-screw extruder (10) according to claim 1 or 2, where the screw (12) has a first pitch value (G1) of a pitch (G) of the screw flights (14) along the conveying zone (18) of the channel (16) and, following in conveying direction (20), has a second pitch value (G2) of the pitch (G) of the screw flights (14) along the conveying zone (18) of the channel (16), where the second pitch value (G2) is larger than or equal to the first pitch value (G1), or alternatively is smaller than the first pitch value (G1).

4. The single-screw extruder (10) according to any of claims 1 to 3, where the screw (12) has, along the channel (16), at least three different pitch values (G1, G2, G3, G4) of the screw flights (14).

5. The single-screw extruder (10) according to any of claims 1 to 4, where the pitch (G) of the screw flights (14) increases along the conveying zone (18) of the channel (16), where the screw flights (14) have a smallest pitch value (G1) along the conveying zone (18) at a transition from the input zone (26) to the conveying zone (18) and a largest pitch value (G2) along the conveying zone (18) at a transition from the conveying zone (18) to the output zone (28) .

6. The single-screw extruder (10) according to any of claims 1 to 5, where the mixing-element arrangement (49) has a pin arrangement (48) comprising at least one pin (48.1, 48.2) extending into an internal space (17) of the channel (16), and has at least one cutout (50) along the screw flights (14), the at least one pin (48.1, 48.2) extending into the at least one cutout (50) of the screw flights (14) when the screw flights (14) are rotated during operation, rotation of the shank (13) of the screw (12) therefore being possible without contact between the pin (48.1, 48.2) and the screw flights (14).

7. The single-screw extruder (10) according to any of claims 1 to 6, with a ratio (d/D) of shank diameter (d) to external screw flight diameter (D) between 0.4 and 0.6.

8. The single-screw extruder (10) according to any of claims 1 to 7, where the screw (12) has, along the channel (16), a smallest pitch value (G1) that is at most between 20% and 85% of a largest pitch value (G4) along the channel (16).

9. The single-screw extruder (10) according to any of claims 1 to 8, where the single-screw extruder (10) is configured to produce an SAP polymer gel (24) with a desired morphology that increases the water absorption of the SAP and/or with an increased throughput, in particular of at least 23 metric tons per hour.

10. A process for changing the morphology of a superabsorbent polymer gel (SAP polymer gel) (24) with a single-screw extruder (10) according to any of claims 1 to 9,
where the process has the following steps:
- introduction of the SAP polymer gel (24) from a polymerization process (38) into the single-screw extruder (10),
- operation of the single-screw extruder (10) in order to change the morphology of the SAP polymer gel (24) and
- discharge of the SAP polymer gel (44) with changed morphology from the single-screw extruder (10) to a drying process (46).

11. The process according to claim 10, where the single-screw extruder (10) has a drive motor (40) and/or a heating device (42), where
- the drive motor (40) is configured to rotate the shank (13) of the screw (12) at up to 150 revolutions per minute, and/or
- the heating device (42) is configured to heat the channel (16) so that the temperature thereof is between 85°C and 140°C, at least in the input zone (26), and during operation of the single-screw extruder (10) the temperature of the SAP polymer gel (24) in the channel (16) increases along the conveying direction (20) as far as an output zone (28).

12. The process according to claim 11, where
- the temperature of the SAP polymer gel (24) in the channel (16) increases along the conveying direction (20) as far as an output zone (28) by a temperature in the range from 20°C to 40°C, and/or
- moreover directly after the output aperture (30) the SAP polymer gel (24) has an again reduced temperature of the again cooled SAP polymer gel (24) between 90°C and 110°C.

13. The use of a single-screw extruder (10) according to any of claims 1 to 9, wherein the single-screw extruder (10) is configured to change a morphology of superabsorbent polymer gel (SAP polymer gel) (24) .

## Revendications

1. Extrudeuse monovis (10) pour modifier une morphologie de polymère superabsorbant, à savoir pour un gel polymère superabsorbant (gel polymère SAP) (24), présentant :
- une ouverture d'entrée (36) pour l'amenée de gel polymère SAP (24),
- un conduit (16) relié à l'ouverture d'entrée (36),
- un arbre à vis sans fin (12) agencé dans le conduit (16) pour le transport et la modification de la morphologie du gel polymère SAP (24), et
- une ouverture de sortie (30) reliée au conduit (16) pour l'évacuation du gel polymère SAP (44) de morphologie modifiée,
l'arbre à vis sans fin (12) présentant un arbre (13) et une vis sans fin (14) agencée sur l'arbre (13) et étant agencé et configuré pour transporter le gel polymère SAP (24) au moyen de la vis sans fin (14) depuis l'ouverture d'entrée (36) jusqu'à l'ouverture de sortie (30),
le conduit (16) présentant une zone d'entrée (26) agencée au niveau ou à proximité de l'ouverture d'entrée (36), une zone de sortie (28) agencée au niveau ou à proximité de l'ouverture de sortie (30) et une zone de transport (18) s'étendant le long du conduit (16) depuis la zone d'entrée (26) jusqu'à la zone de sortie (28),
- l'arbre à vis sans fin (12) ayant une valeur de pas d'un pas (G) de la vis sans fin (14) le long de la zone de transport (18) du conduit (16), et
- le conduit (16) présentant au moins un agencement d'élément de mélange (49) avec au moins un élément de mélange qui fait saillie dans le conduit (16) de l'extrudeuse monovis et qui est configuré pour mélanger le gel polymère SAP (24),
**caractérisée en ce que**
- le conduit (16) présente au moins deux broches (48) agencées en étant séparées l'une de l'autre le long du conduit (16) dans la direction de transport (20) avec un espacement d'élément de mélange (K), une valeur de l'espacement d'élément de mélange (K1, K2, K3, K4) étant inversement adaptée à une valeur de pas (G1, G2, G3) du pas (G) de la vis sans fin (14), à savoir
l'espacement d'élément de mélange (K) est inversement adapté au pas (G) de telle sorte qu'une valeur plus petite de l'espacement d'élément de mélange (K1, K2, K3, K4, K5) est prévue lorsqu'une valeur de pas plus grande (G1, G2, G3) est prévue et une valeur plus grande de l'espacement d'élément de mélange (K1, K2, K3, K4, K5) est prévue lorsqu'une valeur de pas plus petite (G1, G2, G3) est prévue.

2. Extrudeuse monovis (10) selon la revendication 1, dans laquelle l'ouverture de sortie (30) est également configurée pour modifier la morphologie du gel polymère SAP (24).

3. Extrudeuse monovis (10) selon la revendication 1 ou 2, dans laquelle l'arbre à vis sans fin (12) a une première valeur de pas (G1) d'un pas (G) de la vis sans fin (14) le long de la zone de transport (18) du conduit (16) et une deuxième valeur de pas (G2), suivante dans la direction de transport (20), du pas (G) de la vis sans fin (14) le long de la zone de transport (18) du conduit (16), la deuxième valeur de pas (G2) étant supérieure ou égale à la première valeur de pas (G1), ou en variante inférieure à la première valeur de pas (G1).

4. Extrudeuse monovis (10) selon l'une quelconque des revendications 1 à 3, dans laquelle l'arbre à vis sans fin (12) présente au moins trois valeurs de pas différentes (G1, G2, G3, G4) de la vis sans fin (14) le long du conduit (16).

5. Extrudeuse monovis (10) selon l'une quelconque des revendications 1 à 4, dans laquelle le pas (G) de la vis sans fin (14) augmente le long de la zone de transport (18) du conduit (16), la vis sans fin (14) ayant une valeur de pas minimale (G1) le long de la zone de transport (18) au niveau d'une transition de la zone d'entrée (26) à la zone de transport (18) et une valeur de pas maximale (G2) le long de la zone de transport (18) au niveau d'une transition de la zone de transport (18) à la zone de sortie (28).

6. Extrudeuse monovis (10) selon l'une quelconque des revendications 1 à 5, dans laquelle l'agencement d'élément de mélange (49) présente au moins une broche (48.1, 48.2) d'un agencement de broches (48) s'étendant dans un espace intérieur (17) du conduit (16) et au moins un évidement (50) le long de la vis sans fin (14), l'au moins une broche (48.1, 48.2) s'étendant dans l'au moins un évidement (50) de la vis sans fin (14) lorsque la vis sans fin (14) est mise en rotation pendant le fonctionnement, de telle sorte qu'une rotation de l'arbre (13) de l'arbre à vis sans fin (12) est possible sans que la broche (48.1, 48.2) ne touche la vis sans fin (14).

7. Extrudeuse monovis (10) selon l'une quelconque des revendications 1 à 6, avec un rapport (d/D) entre le diamètre de l'arbre (d) et un diamètre extérieur de la vis sans fin (D) compris entre 0,4 et 0,6.

8. Extrudeuse monovis (10) selon l'une quelconque des revendications 1 à 7, dans laquelle l'arbre à vis sans fin (12) a une valeur de pas la plus basse (G1) le long du conduit (16) qui est au plus entre 20 % et 85 % d'une valeur de pas la plus élevée (G4) le long du conduit (16) .

9. Extrudeuse monovis (10) selon l'une quelconque des revendications 1 à 8, l'extrudeuse monovis (10) étant configurée pour produire un gel polymère SAP (24) ayant une morphologie souhaitée augmentant l'absorption d'eau du SAP et/ou avec un débit augmenté, notamment d'au moins 23 tonnes par heure.

10. Procédé pour modifier la morphologie d'un gel polymère superabsorbant (gel polymère SAP) (24) avec une extrudeuse monovis (10) selon l'une quelconque des revendications 1 à 9,
le procédé présentant les étapes consistant à :
- amener le gel polymère SAP (24) à partir d'un procédé de polymérisation (38) dans l'extrudeuse monovis (10),
- faire fonctionner l'extrudeuse monovis (10) afin de modifier la morphologie du gel polymère SAP (24), et
- évacuer le gel polymère SAP (44) de morphologie modifiée de l'extrudeuse monovis (10) vers un procédé de séchage (46).

11. Procédé selon la revendication 10, dans lequel l'extrudeuse monovis (10) présente un moteur d'entraînement (40) et/ou un dispositif de chauffage (42),
- le moteur d'entraînement (40) étant configuré pour mettre en rotation l'arbre (13) de l'arbre à vis sans fin (12) à jusqu'à 150 tours par minute, et/ou
- le dispositif de chauffage (42) étant configuré pour chauffer le conduit (16) de telle sorte que celui-ci ait une température comprise entre 85 °C et 140 °C, en tout cas dans la zone d'entrée (26), et que, lors du fonctionnement de l'extrudeuse monovis (10), la température du gel polymère SAP (24) dans le conduit (16) augmente le long de la direction de transport (20) jusqu'à une zone de sortie (28).

12. Procédé selon la revendication 11, dans lequel
- la température du gel polymère SAP (24) dans le conduit (16) augmente le long de la direction de transport (20) jusqu'à une zone de sortie (28) d'une température dans la plage de 20 °C à 40 °C, et/ou
- directement après l'ouverture de sortie (30), le gel polymère SAP (24) présente une température à nouveau réduite du gel polymère SAP (24) à nouveau refroidi, comprise entre 90 °C et 110 °C.

13. Utilisation d'une extrudeuse monovis (10) selon l'une quelconque des revendications 1 à 9, **caractérisée en ce que** l'extrudeuse monovis (10) est configurée pour modifier une morphologie de gel polymère superabsorbant (gel polymère SAP) (24).
